# EUROPEAN PATENT SPECIFICATION

(11) **EP 0 879 047 B1**
(45) Date of publication and mention of the grant of the patent: **17.07.2002**
(21) Application number: 97903110.1
(22) Date of filing: 24.01.1997
(51) Int. Cl.: A61K 7/50, A61K 7/06

(54) **DETERGENT COMPOSITIONS**
WASCHMITTELZUSAMMENSETZUNGEN
COMPOSITIONS DETERGENTES

(30) Priority: 29.01.1996 US 10784; 03.01.1997 GB 9700059
(43) Date of publication of application: 25.11.1998
(73) Proprietor: Johnson & Johnson Consumer Companies, Inc., Skillman, New Jersey 08558-9418 (US)
(72) Inventor: LUKENBACH, Elvin, R., Flemington, NJ 08822 (US); DOLE, Victoria, F., Whitehouse Station, NJ 08899 (US); NYSTRAND, Glenn, A., Lebanon, NJ 08833 (US); MC CULLOCH, Laura, Basking Ridge, NJ 07920 (US); ALLAN, William, D., Durrington, Worthing BN13 3PU (GB); HILL, Jonathan, R., Lenton, Nottingham NQ7 2EA (GB); TAYLOR, Charles, J., Emsworth, Hampshire PO10 7PU (GB)
(74) Representative: Mercer, Christopher Paul
(86) International application number: US9701196
(87) International publication number: WO9726860

(56) References cited:
- EP-A- 0 127 580
- EP-A- 0 416 447
- EP-A- 0 463 780
- GB-A- 2 283 755

## Description

### Background of the Invention

### 1. Field of the Invention

This invention relates to conditioning detergent compositions suitable for use in personal cleansing application which not only impart cleansing, wet detangling, dry detangling and manageability properties to hair, but also which are relatively non-irritating and thus suitable for use by young children and adults having sensitive skin and eyes.

### 2. Description of the Prior Art

In the past, it has been considered desirable to cleanse hair and then to condition it after cleansing. For many years, it was necessary to perform these acts in two separate steps. However, with the advent of so-called "two-in-one" conditioning shampoos, it became possible to condition and cleanse simultaneously. Unfortunately, many of these two-in-one conditioning shampoos and body cleansers have proven to be relatively irritating to the eyes and skin and uncomfortable for use with children or sensitive adults. Therefore, it is an object of this invention to create a conditioning shampoo which has good cleansing ability, excellent conditioning properties and has a low degree of ocular and skin irritation.

### Summary of the Invention

In accordance with this invention, there is provided a detergent composition comprising:
a surfactant portion comprising:
   1. a nonionic surfactant;
   2. an amphoteric surfactant; and
   3. an anionic surfactant; and
a conditioner portion comprising at least two cationic conditioning polymers selected from:
   1. a cationic cellulose derivative;
   2. a cationic guar derivative; and
   3. a homopolymer or copolymer of a cationic monomer selected from:
      a. a monomer having the formula 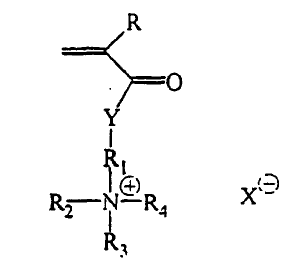 wherein
         R is H or CH₃,
         Y is O or NH,
         R₁ is an alkylene group having from about 2 to about 6 carbon atoms,
         R₂, R₃ and R₄ are each independently an alkyl group or hydroxyalkyl group having from about 1 to about 22 carbon atoms, and
         X is a monovalent anion selected from halide and alkyl sulfate having from about 1 to about 4 carbon atoms, or
      b. diallyldimethylammonium chloride.

In accordance with another embodiment of this invention, there is provided a detergent composition comprising, based upon the total weight of the composition:
a. a carboxyalkyl alkylpolyamine amphoteric surfactant of the formula: 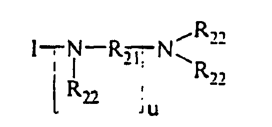 wherein
   I is an alkyl or alkenyl group containing from about 8 to about 22 carbon atoms;
   R₂₂ is a carboxyalkyl group having from about 2 to about 3 carbon atoms;
   R₂₁ is an alkylene group having from about 2 to about 3 carbon atoms
   u is an integer of 1 to 4; and
b. an anionic surfactant, except those anionic surfactants of the group consisting of
   1) an alkyl sulfate of the formula

      R'-CH₂OSO₃X';

      and
   2) an alkylaryl sulfonate of the formula 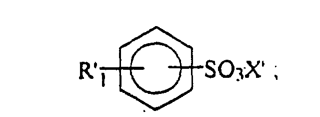 wherein
      R' is an alkyl group having from about 7 to about 14 carbon atoms,
      R'₁ is an alkyl group having from about 1 to about 12 carbon atoms,
      X' is selected from the group consisting of alkali metal ions, alkaline earth metal ions, ammonium ions, and ammonium ions substituted with from about 1 to about 3 substituents; each of the substituents may be the same or different and are selected from the group consisting of alkyl groups having 1 to 4 carbon atoms and hydroxyalkyl groups having from about 2 to about 4 carbon atoms; and
c.) optionally a non-ionic surfactant,
with the proviso that if the non-ionic surfactant is omitted and the anionic surfactant is an alkyl ether sulfate of the formula

R'(OCH₂CH₂)ᵥOSO₃X',

then v is greater than or equal to 3.

In accordance with yet another embodiment of this invention, there is provided a detergent composition comprising
a. an amidoalkyl sultaine amphoteric surfactant of the formula: 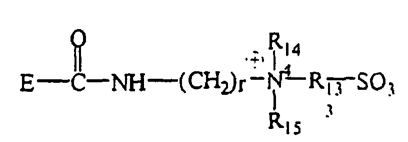 wherein
   E is an alkyl group or alkenyl group having from about 7 to about 21 carbon atoms;
   R₁₄ and R₁₅ are each independently an alkyl group or a hydroxyalkyl group having from about 1 to about 4 carbon atoms;
   r is an integer from about 2 to about 6; and
   R₁₃ is an alkylene or hydroxyalkylene group having from about 2 to about 3 carbon atoms;
b. an anionic surfactant, except those anionic surfactants of the group consisting of
   1. an alkyl sulfate of the formula

      R'-CH₂OSO₃X';
   2. an alkyl ether sulfate of the formula

      R'(OCH₂CH₂)ᵥOSO₃X';

      and
   3. an alkylaryl sulfonate of the formula 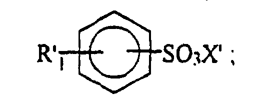 wherein
      R' is an alkyl group having from about 7 to about 14 carbon atoms,
      R'₁ is an alkyl group having from about 1 to about 12 carbon atoms,
      X' is selected from the group consisting of alkali metal ions, alkaline earth metal ions, and ammonium ions, and ammonium ions substituted with from about 1 to about 3 substituents; each of the substituents may be the same or different and are selected from the group consisting of alkyl groups having 1 to 4 carbon atoms and hydroxyalkyl groups having from about 2 to about 4 carbon atoms; and
      v is an integer from 1 to 5; and
c. optionally a non-ionic surfactant.

The composition of this invention, when used in a shampoo or body cleanser, possesses one or more of the following properties: cleansing, wet detangling, dry detangling, manageability, and low degree of ocular irritation.

### Detailed Description of Preferred Embodiments

In one embodiment of the present invention, the shampoo composition may suitably comprise, consist of, or consist essentially of an anionic surfactant, an amphoteric surfactant, a non-ionic surfactant, and at least two cationic conditioning polymers. The composition is preferably comprised of, based upon the total weight of the shampoo composition, from about 5 percent to about 20 percent, and more preferably from about 5 percent to about 14 percent of a surfactant portion and, based upon the total weight of the composition, from about 0.01 percent to about 3.0 percent, preferably from about 0.01 percent to about 2.0 percent, more preferably from about 0.01 percent to about 1.0 percent, even more preferably from about 0.01 percent to about 0.5 percent, and most preferably from about 0.01 percent to about 0.3 percent, of a conditioner portion.

In this embodiment, the surfactant portion of the present invention contains nonionic, amphoteric and anionic surfactants. Preferably the weight ratio between the amphoteric surfactant and the anionic surfactant may range from about 3:1 to about 1:3, and preferably from about 2:1 to about 1:2. The weight ratio of the amphoteric/anionic surfactant combination:non-ionic surfactant may vary widely, and preferably is about 2:1 to about 1:2. The nonionic surfactant is present in an amount, based upon the total weight of the shampoo composition, of from about 0.1 percent to about 10 percent, preferably from about 1 percent to about 10 percent, and more preferably from about 4 percent to about 8 percent. The amphoteric surfactant is present in an amount, based upon the total weight of the shampoo composition, of from about 0.5 percent to about 10 percent, preferably from about 1 percent to about 8 percent, and more preferably from about 2 percent to about 6 percent. The anionic surfactant is present in the shampoo composition in an amount from about 1.0 percent to about 10 percent, preferably from about 1 percent to about 8 percent, and more preferably from about 1 percent to about 6 percent, based on the overall weight of the shampoo composition.
One class of nonionic surfactants usefu, in the present invention are polyoxyethylene derivatives of polyol esters, wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerine, pentaerythritol and mixtures thereof, (2) contains an average of from about 10 to about 120, and preferably about 20 to about 80 oxyethylene units: and (3) has an average of about 1 to about 3 fatty acid residues per mole of polyoxyethylene derivative of polyol ester.

Examples of preferred polyoxyethylene derivatives of polyol esters include, but are not limited to PEG-80 sorbitan laurate and Polysorbate 20. PEG-80 sorbitan laurate, which is a sorbitan monoester of lauric add ethoxylated with an average of about 80 moles of ethylene oxide, is available commercially from ICI Surfactants of Wilmington, Delaware under the tradename, Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Delaware under the tradename "Tween 20".

Another class of suitable nonionic surfactants includes long chain alkyl glucosides or polyglucosides, which are the condensation products of (a) a long chain alcohol containing from about 6 to about 22, and preferably from about 8 to about 14 carbon atoms, with (b) glucose or a glucose-containing polymer. The alkyl gluocosides have about 1 to about 6 glucose residues per molecule of alkyl glucoside. A preferred glucoside is decyl glucoside, which is the condensation product of decyl alcohol with a glucose polymer and is available commercially from Henkel Corporation of Hoboken, New Jersey under the tradename, "Plantaren 2000."

The compositions of the present invention also contain an amphoteric surfactant. As used herein, the term "amphoteric" shall mean: 1) molecules that contain both acidic and basic sites such as, for example, an amino add containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. Examples of zwitterionic materials include, but are not limited to, alkyl betaines and amidoalkyl betaines. The amphoteric surfactants are disclosed herein without a counter ion. One skilled in the art would readily recognize that under the pH conditions of the compositions of the present invention. the amphoteric surfactants are either electrically neutral by virtue of having balancing positive and negative charges, or they have counter ions such as alkali metal, alkaline earth, or ammonium counter ions.

Commercially available amphoteric surfactants are suitable for use in the present invention and include, but are not limited to amphocarboxylates, alkyl betaines, amidoalkyl betaines, amidoalkyl sultaines, amphophosphates, phosphobetaines, pyrophosphobetaines, carboxyalkyl alkyl polyamines and mixtures thereof.

Examples of suitable amphocarboxylate compounds include those of the formula:

A-CONH(CH₂)ₓN⁺R₅R₆R₇

wherein
A is an alkyl or alkenyl group having from about 7 to about 21, and preferably from about 10 to about 16 carbon atoms;
x is an integer of from about 2 to about 6;
R₅ is hydrogen or a carboxyalkyl group containing from about 2 to about 3 carbon atoms, and preferably is hydrogen;
R₆ is a hydroxyalkyl group containing from about 2 to about 3 carbon atoms or is a group of the formula:

   R₈-O-(CH₂)ₙCO₂

   wherein
   R₈ is an alkylene group having from about 2 to about 3 carbon atoms and n is 1 or 2; and
R₇ is a carboxyalkyl group containing from about 2 to about 3 carbon atoms;
Preferably, the amphocarboxylate compound is an imidazoline surfactant, and more preferably a disodium lauroamphodiacetate, which is commercially available from Mona Chemical Company of Paterson. New Jersey under the tradename, "Monateric 949J." When an amphocarboxylate is used in the shampoo composition, it should be present in an amount of about 0.5 percent to about 10 percent, and preferably from about 0.5 percent to about 6 percent, based on the overall weight of the composition.

Examples of suitable alkyl betaines include those compounds of the formula:

B-N⁺R₉R₁₀(CH₂)ₚCO₂⁻

wherein
B is an alkyl or alkenyl group having from about 8 to about 22, and preferably from about 8 to about 16 carbon atoms;
R₉ and R₁₀ are each independently an alkyl or hydroxyalkyl group having from about 1 to about 4 carbon atoms; and
p is 1 or 2.
A preferred betaine for use in the present invention is lauryl betaine, available commercially from Albright & Wilson, Ltd. of West Midlands, United Kingdom as "Empigen BB/J." If present, the alkyl betaine should be used in an amount, based on the overall weight of the composition, of from about 0.25 percent to about 10 percent, preferably from about 025 percent to about 8 percent, and more preferably, from about 0.25 percent to about 5 percent.

Examples of suitable amidoalkyl betaines include those compounds of the formula:

D-CO-NH(CH₂)_{q}-N⁺R₁₁R₁₂(CH₂)ₘCO₂⁻

wherein
D is an alkyl or alkenyl group having from about 7 to about 21, and preferably from about 7 to about 15 carbon atoms;
R₁₁ and R₁₂ are each independently an alkyl or hydroxyalkyl group having from about 1 to about 4 carbon atoms;
q is an integer from about 2 to about 6; and m is 1 or 2.
A preferred amidoalkyl betaine is cocamidopropyl betaine, available commercially from Goldschmidt Chemical Corporation of Hopewell, Virginia under the tradename, "Tegobetaine L7." When present in the shampoo compositions of this invention, the amidoalkyl betaine should be used in an amount of from about 0.25 percent to about 10 percent, preferably from about 0.25 percent to about 8 percent, and more preferably from about 0.25 percent to about 5 percent, based on the overall weight of the composition.

Examples of suitable amidoalkyl sultaines include those compounds of the formula 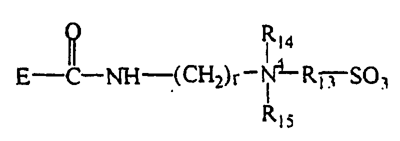 wherein
E is an alkyl or alkenyl group having from about 7 to about 21, and preferably from about 7 to about 15 carbon atoms;
R₁₄ and R₁₅ are each independently an alkyl, or hydroxyalkyl group having from about 1 to about 4 carbon atoms;
r is an integer from about 2 to about 6; and
R₁₃ is an alkylene or hydroxyalkylene group having from about 2 to about 3 carbon atoms;

Preferably the amidoalkyl sultaine is cocamidopropyl hydroxysultaine, available commercially from Rhone-Poulenc Inc. of Cranbury, New Jersey under the tradename, "Mirataine CBS." When present in the shampoo compositions of this invention, it should be used in an amount of from about 0.5 percent to about 10 percent, preferably from about 1.0 percent to about 6 percent, and more preferably from about 1.5 percent to about 5 percent, based on the overall weight of the composition.

Examples of suitable amphophosphate compounds include those of the formula: 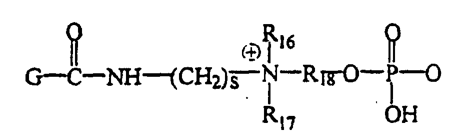 wherein
G is an alkyl or alkenyl group having about 7 to about 21, and preferably from about 7 to about 15 carbon atoms;
s is an integer from about 2 to about 6;
R₁₆ is hydrogen or a carboxyalkyl group containing from about 2 to about 3 carbon atoms;
R₁₇ is a hydroxyalkyl group containing from about 2 to about 3 carbon atoms or a group of the formula:

   R₁₉-O-(CH₂)ₜ-CO₂

   wherein
   R₁₉ is an alkylene or hydroxyalkylene group having from about 2 to about 3 carbon atoms and
   t is 1 or 2; and
R₁₈ is an alkylene or hydroxyalkylene group having from about 2 to about 3 carbon atoms.

Preferably the amphophosphate compounds are sodium lauroampho PG-acetate phosphate, available commercially from Mona Industries of Paterson, New Jersey under the tradename, "Monateric 1023," and those disclosed in U.S. Patent 4,380,637, with sodium lauroampho PG-acetate phosphate being most preferred.

Examples of suitable phosphobetaines include those compounds of the formula: 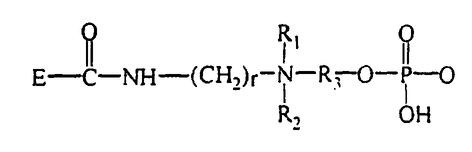 wherein E, r, R₁, R₂ and R₃, are as defined above. Preferably the phosphobetaine compounds are those disclosed in U.S. Patent Nos. 4,215,064,4,617,414, and 4,233,192.

Examples of suitable pyrophosphobetaines include those compounds of the formula: 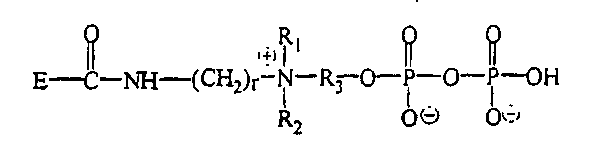 wherein E, r, R₁, R₂ and R₃, are as defined above. Preferably the pyrophosphobetaine compounds are those disclosed in U.S. Patent Nos. 4,382,036, 4,372,869, and 4,617,414.

Examples of suitable carboxyalkyl alkylpolyamines include those of the formula: 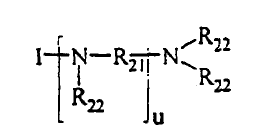 wherein
I is an alkyl or alkenyl group containing from about 8 to about 22, and preferably from about 8 to about 16 carbon atoms;
R₂₂ is a carboxyalkyl group having from about 2 to about 3 carbon atoms;
R₂₁ is an alkylene group having from about 2 to about 3 carbon atoms and
u is an integer from about 1 to about 4.

Preferably the carboxyalkyl alkyl polyamine is sodium carboxymethyl coco polypropylamme, available commercially from Akzo Nobel Surface Chemistry under the tradename, "Ampholak 7CX/C." When present in the shampoo compositions of this invention, it should be used in an amount of from about 0. 5 percent to about 10 percent, preferably from about 1.0 percent to about 8 percent, and more preferably from about 2.0 percent to about 6.0 percent, based on the overall weight of the composition.

In a preferred embodiment, the amphoteric surfactant portion of the compositions is comprised of a mixture of amphoteric surfactants, such as amphocarboxylate and alkyl betaine. or amphocarboxylate and amidoalkyl betaine. In this embodiment, the amphocarboxylate is present in the shampoo composition in an amount, based upon the total weight of the shampoo composition, of from about 0.5 percent to about 9.5 percent and the alkyl betaine or amidoalkyl betaine is present in an amount, based upon the total weight of the shampoo composition, of from about 9.5 percent to about 0.5 percent.

The compositions of this embodiment also contain at least one anionic surfactant. Preferably, the anionic surfactant is selected from the following classes of surfactants:
an alkyl sulfate of the formula

   R'-CH₂OSO₃X';
an alkyl ether sulfate of the formula

   R'(OCH₂CH₂)ᵥOSO₃X';
an alkyl monoglyceryl ether sulfate of the formula 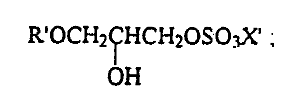
an alkyl monoglyceride sulfate of the formula 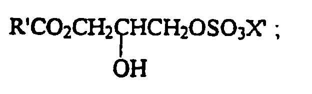
an alkyl monoglyceride sulfonate of the formula 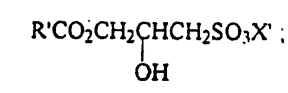
an alkyl sulfonate of the formula

   R'-SO₃X';
an alkylaryl sulfonate of the formula 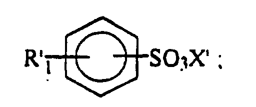
an alkyl sulfosuccinate of the formula: 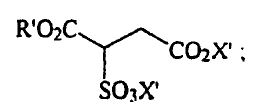
an alkyl ether sulfosuccinate of the formula: 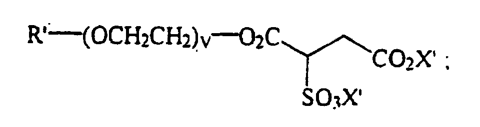
an alkyl sulfosuccinamate of the formula: 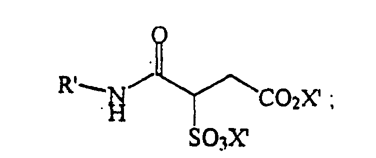
an alkyl amidosulfosuccinate of the formula 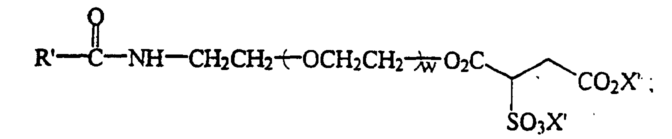
an alkyl carboxylate of the formula:

   R¹―(OCH₂CH₂)_{w}-OCH₂CO₂X';
an alkyl amidoethercarboxylate of the formula: 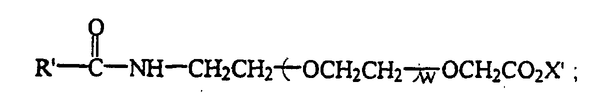
an alkyl succinate of the formula: 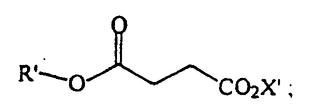
a fatty acyl sarcosinate of the formula: 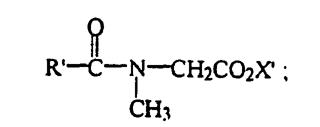
a fatty acyl amino acid of the formula: 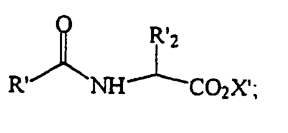
*** a fatty acyl taurate of the formula: 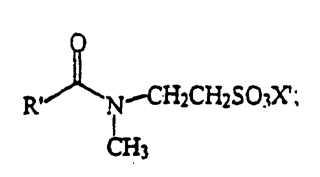
a fatty alkyl sulfoacetate of the formula: 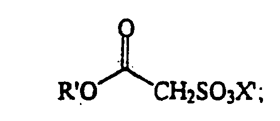 wherein
   R' is an alkyl group having from about 7 to about 22, and preferably fom about 7 to about 16 carbon atoms,
   R'₁ is an alkyl group having from about 1 to about 18, and preferably from about 8 to about 14 carbon atoms,
   R'₂ is a substituent of a natural or synthetic I-amino acid.
   X' is selected from the group consisting of alkali metal ions, alkaline earth metal ions, ammonium ions, and ammonium ions substituted with from about 1 to about 3 substituents, each of the substituents may be the same or different and are selected from the group consisting of alkyl groups having from 1 to 4 carbon atoms and hydroxyalkyl groups having from about 2 to about 4 carbon atoms and
   v is an integer from 1 to 6;
   w is an integer from 0 to 20;
and mixtures thereof. Preferably the anionic surfactant is comprised of sodium trideceth sutfate, sodium laureth sulfate, disodium laureth sulfosuccinate, or mixtures thereof. Sodium trideceth sulfate is the sodium salt of sulfated ethoxylated tridecyl, alcohol that conforms generally to the following formula C₁₃H₂₇(OCH₂CH₂)ₙOSO₃Na, where n has a value between 1 and 4, and is commercially available from Stepan Company of Northfield, Illinois under the tradename, "Cedapal TD-403M." Sodium laureth sulfate is available from from Albright & Wilson, Ltd. West Midlands, United Kingdom under the tradename, "Empicol 0251/70-J." Disodium laureth sulfosuccinate is available commercially from Albright & Wilson, Ltd. of West Midlands, United Kingdom under the tradename, "Empicol SDD."

In this embodiment, the shampoo composition of the present invention also contains a conditioner portion which is comprised of at least two cationic conditioning polymers. Preferred cationic conditioning polymers are selected from the following:
1. a cationic cellulose derivative;
2. a cationic guar derivative; and
3. a homopolymer or copolymer of a cationic monomer selected from:
   a. a monomer having formula I. 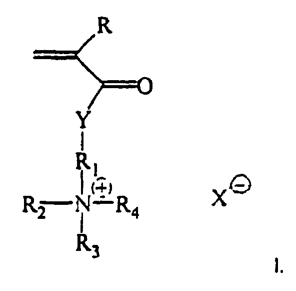 wherein
      R is H or CH₃,
      Y is O or NH,
      R₁ is an alkylene group having from about 2 to about 6, and preferably from about 2 to about 3 carbon atoms,
      R₂, R₃ and R₄ are each independently an alkyl group having from about 1 to about 22, and preferably from about 1 to about 4 carbon atoms, and
      X is a monovalent anion selected from halide and alkyl sulfate, or
   b. diallyldimethylammonium chloride.

The amount of each conditioner component may range, based upon the total weight of the composition, from about 0.01 percent to about 1.0 percent, preferably from about 0.01 percent to about 0.5 percent, and more preferably from about 0.01 to about 0.2 percent.

Preferably, the cationic cellulose derivative is a polymeric quaternary ammonium salt derived from the reaction of hydroxyethyl cellulose with a trimethylammonium substituted epoxide. The material known as Polyquatemium-10, commercially available from Amerchol Corporation of Edison, New Jersey as "Polymer JR-400," is especially useful in this regard.

The cationic guar derivative is preferably a guar hydroxypropyltrimonium chloride, available commercially from Rhone-Poulenc Inc., of Cranbury, New Jersey under the tradename, "Jaguar C-17."

Another preferred cationic polymer includes those compounds derived from acrylamidopropyl trimonium chloride which has the formula: 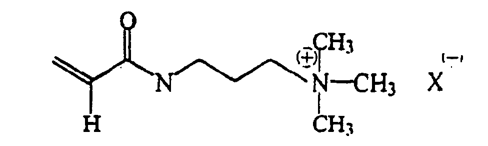 and more preferably is the copolymer of this monomer with acrylamide, the latter of which is available commercially from Allied Colloids, of Suffolk, Virginia under the tradename, "Salcare SC60."

Other preferred cationic conditioning polymers are those derived from the monomer diallyldimethylammonium chloride. The homopolymer of this monomer is Polyquaternium-6, which is available commercially form Allied Colloids of Suffolk, Virginia under the tradename, "Salcare SC30." The copolymer of diallyldimethylammonium chloride with acrylamide is known as Polyquaternium-7, and is also available from Allied Colloids under the tradename "Salcare SC10."

In a preferred embodiment, the conditioner portion is a combination of cationic cellulose derivative with a cationic guar derivative. In this embodiment, the cationic cellulose derivative is present in the composition in an amount, based on the overall weight of the shampoo composition, of from about 0.01 percent to about 2 percent, preferably from about 0.05 percent to about 1.0 percent, and more preferably from about 0.05 percent to about 0.3 percent, and the cationic guar derivative is present in an amount, based on the overall weight of the shampoo composition, of from about 0.01 percent to about 1.0 percent, preferably from about 0.05 percent to about 1.0 percent, and more preferably from about 0.05 percent to about 0.3 percent.

In another preferred embodiment, the conditioner portion is comprised of cationic cellulose derivative or cationic guar derivative and a homopolymer or copolymer of the cationic monomer having formula I. In this embodiment, the cationic cellulose derivative or cationic guar derivative is present in an amount, based on the overall weight of the composition, of from about 0.01 percent to about 0.5 percent, and preferably from about 0.01 percent to about 0.2 percent, and the homopolymer or copolymer of the above monomer is present in an amount, based on the overall weight of the composition, of from about 0.01 percent to about 0.5 percent, preferably from about 0.01 percent to about 0.2 percent.

In another preferred embodiment, the conditioner portion is comprised of cationic guar derivative and a homopolymer or copolymer of diallyldimethylammonium chloride. In this embodiment, the cationic guar derivative is present in an amount, based on the overall weight of the shampoo composition, of from about, 0.01 percent to about 0.5 percent, preferably from about 0.01 percent to about 0.2 percent, and the homopolymer or copolymer of diallyldimethylammonium chloride is present in an amount, based on the overall weight of the shampoo composition, of from about 0.01 percent to about 0.5 percent, preferably from about 0.01 percent to about 0.2 percent.

In accordance with another embodiment of this invention, there is provided a composition suitably comprised of, consisting of, or consisting essentially of an amphoteric surfactant and an anionic surfactant, with the total amount of surfactants ranging, based upon the total weight of the composition, from about 4 percent to about 20 percent, preferably from about 4 percent to about 15 percent, and more preferably from about 4 percent to about 10 percent. Examples of suitable amphoteric surfactants include those described above and preferably include the above-described carboxyalkyl alkylpolyamines, the amidoalkyl sultaines, and mixtures thereof. Examples of suitable anionic surfactants include those described above and preferably include those anionic surfactants except the anionic surfactant compounds of the group consisting of: 1) the above-described alkyl sulfates or alkylaryl sulfonates when the amphoteric surfactant is the above described carboxyalkyl alkylpolyamine; and 2) the alkyl sulfates, alkyl ether sulfates, and alkylaryl sulfonates when the amphoteric surfactant is the above-described amidoalkyl sultaine.

In this embodiment, the amount of each of the amphoteric surfactant and anionic surfactant used in the composition may range, based upon the total weight of the composition, from about 2 percent to about 10 percent, and preferably from about 2 percent to about 6 percent, respectively. The weight ratio of amphoteric surfactant:anionic surfactant may range from about 3:1 to about 1:3, preferably from about 2:1 to about 1:2, and most preferably from about 1.5:1 to about 1:1.5. Optionally, the composition of this embodiment may contain one or more of the above-mentioned non-ionic surfactants and/or one or more of the above-mentioned cationic conditioners. Preferably, the non-ionic surfactant, if used, is a polyoxyethylene derivative of a polyol ester, more preferably Polysorbate 20, and the preferred cationic conditioner is Polyquarternium 10, guar hydroxypropyltriammonium chloride, acrylamidopropyl trimonium chloride/acrylamide copolymer, and mixtures thereof. The amount of nonionic surfactant used in the composition may range, based upon the total weight of the composition, of from about 0 to about 5 percent, and preferably from about 0.5 percent to about 1 percent. When the nonionic surfactant is used, the weight ratio of amphoteric/anionic surfactant:nonionic surfactant is from about 40:1 to about 2:1 and preferably from about 20:1 to about 10:1. The amount of each cationic conditioner used in the composition may range, based upon the total weight of the composition, from about 0 to about 0.5 percent, and preferably from greater than about 0 percent to about 0.3 percent, and more preferably from greater than about 0 percent to about 0.2 percent.

The composition of the present invention may also include one or more optional ingredients nonexclusively including a pearlescent or opacifying agent, a thickening agent, secondary conditioners, humectants, chelating agents, and additives which enhance their appearance, feel and fragrance, such as colorants, fragrances, preservatives, pH adjusting agents, and the like. The pH of the shampoo compositions of this invention is preferably maintained in the range of from about 5 to about 7.5, and more preferably from about 5.5 to about 7.0.

Commercially available pearlescent or opacifying agents which are capable of suspending water insoluble additives such as silicones and/or which tend to indicate to consumers that the resultant product is a conditioning shampoo are suitable for use in this invention. The pearlescent or opacifying agent is present in an amount, based upon the total weight of the composition, of from about 0 percent to about 3 percent, preferably from about 0.25 percent to about 2.5 percent, and more preferably, from about 0.5 percent to about 1.5 percent. Examples of suitable peariescent or opacifying agents include, but are not limited to mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula

HO-(JO)ₐ-H

wherein
J is an alkylene group having from about 2 to about 3 carbon atoms; and a is 2 or 3;
fatty alcohols containing from about 16 to about 22 carbon atoms; fatty esters of the formula

KCOOCH₂L

wherein K and L independently contain from about 15 to about 21 carbon atoms;
inorganic solids insoluble in the shampoo composition, and mixtures thereof.

In a preferred embodiment, the pearlescent or opacifying agent is introduced to the shampoo composition as a pre-formed, stabilized aqueous dispersion, such as that commercially available from Henkel Corporation of Hoboken, New Jersey under the tradename, "Euperlan PK-3000." This material is a combination of glycol distearate (the diester of ethylene glycol and stearic acid), Laureth-4 (CH₃(CH₂)₁₀CH₂(OCH₂CH₂)₄OH) and cocamidopropyl betaine and preferably is in a weight percent ratio of from about 25 to about 30: about 3 to about 15: about 20 to about 25, respectively.

Commercially available thickening agents which are capable of imparting the appropriate viscosity to the conditioning shampoo compositions are suitable for use in this invention. If used, the thickener should be present in the shampoo compositions in an amount sufficient to raise the Brookfield viscosity of the composition to a value of between about 500 to about 10,000 centipoise. Examples of suitable thickening agents nonexdusively include: mono or diesters of 1) polyethylene glycol of formula

HO-(CH₂CH₂O)_{z}H

wherein z is an integer from about 3 to about 200;
and 2) fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; and mixtures thereof. Preferred thickeners include polyethylene glycol ester, and more preferably PEG-150 distearate which is available from the Stepan Company of Northfield, Illinois or from Corniel, S.p.A. of Bologna, Italy under the tradename, "PEG 6000 DS."

Commercially available secondary conditioners such as volatile silicones which imparts additional attributes such as gloss to the hair are suitable for use in this invention. Preferably, the volatile silicone conditioning agent has an atmospheric pressure boiling point less than about 220 C. The volatile silicone conditioner is present in an amount of from about 0 percent to about 3 percent, preferably from about 0.25 percent to about 2.5 percent, and more preferably from about 0.5 percent to about 1.0 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Coming Corporation of Midland, Michigan under the tradename. "DC-345" and mixtures thereof, and preferably include cyclomethicone fluids.

Commercially available humectants which are capable of providing moisturization and conditioning properties to the shampoo composition are suitable for use in the present invention. The humectant is present in an amount of from about 0 percent to about 10 percent, preferably from about 0.5 percent to about 5 percent, and more preferably from about 0.5 percent to about 3 percent, based on the overall weight of the composition. Examples of suitable humectants nonexclusively include: 1) water soluble liquid polyols selected from the group comprising glycerine, propylene glycol, hexylene glycol butylene glycol, dipropylene glycol, and mixtures thereof; 2)polyalkylene glycol of the formula

HO-(RO)_{b}-H

wherein R" is an alkylene group having from about 2 to about 3 carbon atoms and b is an integer of from about 2 to about 10;
3) polyethylene glycol ether of methyl glucose of formula

   CH₃-C₆H₁₀O₅-(OCH₂CH₂)_{c}-OH

   wherein c is an integer from about 5 to about 25;
4) urea; and 5) mixtures thereof, with glycerine being the preferred humectant.

Examples of suitable chelating agents include those which are capable of protecting and preserving the compositions of this invention. Preferably, the chelating agent is EDTA, and more preferably is tetrasodium EDTA available commercially from Dow Chemical Company of Midland. Michigan under the tradename, "Versene 100XL" and is present in an amount, based upon the total weight of the composition, from about 0 to about 0.5 percent, and preferably from about 0.05 percent to about 0.25 percent. Suitable preservatives include Quaternium 15, available commercially as Dowicil 200" from the Dow Chemical Corporation of Midland, Michigan, and are present in the composition in an amount, based upon the total weight of the composition, from about 0 to about 0.2 percent, and preferably from about 0.05 percent to about 0.10 percent.

The above described composition may be prepared by combining the desired components in a suitable container and mixing them under ambient conditions in any conventional mixing means well known in the art, such as a mechanically stirred propeller, paddle, and the like. Although the order of mixing is not critical, it is preferable to pre-blend certain components, such as the fragrance and the nonionic surfactant before adding such components into the main mixture.

When a cationic guar conditioner is used, it is also preferable to preblend the cationic guar conditioner with glycerin under ambient conditions, then allow the guar conditioner to be wet-out"by the glycerin. Although the time to wet-out" may vary, typically this time period may range from about 5 minutes to about 30 minutes. Preferably, the guar conditioner:glycerin weight ratio is from about 1:100 to about 1:1, and more preferably from about 1:50 to about 1:5, and most preferably from about 1:15 to about 1:7. The resulting suspension is mixed with water under ambient conditions at a suspension:water weight ratio of from about 1:5 to about 1:20. The resulting water-suspension mixture is then acidified with an amount of acid, preferably citric acid, effective to reduce the pH of the overall composition to a value of about 4.

When using a thickener component, it is also preferable to preblend the desired thickener with from about 5 percent to about 20 percent, based upon the total weight of the composition, of water and preferably at a temperature of from about 60°C to about 80°C. When processing with a thickener, it is also preferable to reduce the temperature of the overall composition to less than about 45°C before any preformed pearlizer is added thereto.

The detergent composition of the present invention is preferably used in personal cleansing applications nonexclusively including shampoos, gels such as shower gels, baths such as baby baths, and the like.

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### Examples

All amounts of materials are given in parts by weight based on 100 parts of the overall formulation unless stated otherwise. The following test procedures were used in the following Examples:
**1. Hair Conditioning Properties:** Conditioning properties of shampoos are determined by measuring the average energy and force required to comb hair in the wet and dry state after the hair has been washed with a particular shampoo formulation in accordance with the method set forth as follows:
   **a) *Preparation of Hair samples:*** Human hair tresses are prepared by weighing out about 10-12 grams of virgin brown hair, and binding the cuticle end with a cable tie and hot melt glue. The cuticle end of the bundle is positioned in a binder clip. The hair is fanned out evenly over the width of the binder clip. Hot melt glue is applied along the edge of the binder clip, joining the clip and the hair. Glue is applied to the inside of the clip for further strength. A rubber band is applied to the outside of the clip, to keep the jaws of the clip from separating. The glue is allowed to dry thoroughly. The tress is washed to remove contaminants such as dust or shampoo residue by immersing the tress in methanol for ten seconds and removing the tress and allowing it to air dry. Loose hair is removed. Tangles are removed by combing the tresses with a standard comb or brush. Static charge buildup is removed using a static reducing gun.
      The number of trials required for the test is equal to the number of formulations (and suitable controls) under test. The formulations are randomized such that each product is applied to each tress at some point in time. Two shampooings each using about 1 cc of shampoo composition are required. The tress is thoroughly wet under running, 100 F tap water. About 1 cc of a given shampoo composition is applied evenly from top to bottom of the tress. Using the fingers of both hands, the shampoo is rubbed into the hair for approximately 30 seconds to produce lather. The tress is then rinsed thoroughly under running, 100°F water. The tress is then again washed and rinsed using a second 1 cc sample of product. The tress is then allowed to drip dry for 5 minutes.
      The tresses are then suspended from a sturdy ring stand such that they hang freely and have several inches of clearance between the bottom of the tress and the top of the bench.
   **b) *Wet Detangling Energy:*** A Combing Force Device (CFD), which is a hand held, electromechanical instrument which measures the amount of force or energy required to pass a comb through the hair, is held horizontally in the one hand and tangles are removed from the tresses by starting at the lower portion of the tress and moving the CFD downward. Each successive stroke is started at a point which is higher than the previous stroke. This measurement continues until the CFD passes freely through the entire length of the tress. Once all tangles have been removed, three top-to-bottom strokes complete the detangling measurement. The cumulative energy to detangle the hair tresses is reported as wet detangling energy, in units of gram-seconds (g/sec).
   **c) *Wet Comb Force:*** After the detangling energy measurement is completed on all tresses, the tresses are measured for wet comb force. A sensor attached to a curling iron measures the twisting, or torsional force of the curling iron as the instrument is moved though the hair. The instrument is passed through the detangled tresses about 25 times. Comb force, expressed in grams, is the median force required to pass the comb through the detangled tress.
   d) ***Dry Detangling Energy*:** After the tresses are blow-dried until they are no longer damp, the detangling procedure set forth in b is repeated using the dry tresses.
   **e) *Dry Comb Force:*** After the tresses are blow-dried until they are no longer damp and dry detangling energy is determined, the combing procedure set forth in c) is repeated using the dry tresses.
**2.) Ocular Irritation Properties:** Irritation to the eyes expected for a given formulation is measured in accordance with the Invittox Protocol Number 86, the "Trans-epithelial Permeability (TEP) Assay" as set forth in Invittox Protocol Number 86 (May 1994). In general, the ocular irritation potential of a product can be evaluated by determining its effect on the permeability of a cell layer, as assessed by the leakage of fluorescein through the layer. Monolayers of Madin-Darby canine kidney (MDCK) cells are grown to confluence on microporous inserts in a 24-well plate containing medium or assay buffer in the lower wells. The irritation potential of a product is evaluated by measuring the damage to the permeability barrier in the cell monolayer following a 15 minute exposure to dilutions of the product. Barrier damage is assessed by the amount of sodium fluorescein that has leaked through to the lower well after 30 minutes, as determined spectrophotometrically. The fluorescein leakage is plotted against the concentration of test material to determine the EC₅₀ (the concentration of test material that causes 50% of maximum dye leakage, i.e.. 50% damage to the permeability barrier).

Exposure of a layer of MDCK cells grown on a microporous membrane to a test sample is a model for the first event that occurs when an irritant comes in contact with the eye. In vivo, the outermost layers of the corneal epithelium form a selectively permeable barrier due to the presence of tight junctions between cells. On exposure to an irritant, the tight junctions separate, thereby removing the permeability barrier. Fluid is imbibed to the underlying layers of epithelium and to the stroma, causing the collagen lamellae to separate, resulting in opacity. The TEP assay measures the effect of an irritant on the breakdown of tight junctions between cells in a layer of MDCK cells grown on a microporous insert. Damage is evaluated spectrophotometrically, by measuring the amount of marker dye (sodium fluorescein) that leaks through the cell layer and microporous membrane to the lower well. Generally, a passing score is reflected in an EC₅₀ of 2.2% or higher.

### Example 1: Compounding of Shampoo Composition

The following pre-blends were prepared:
Preblend A: 1.5 parts of PEG 6000 DS were mixed with 20 parts deionized water at 65°C in a mixing vessel until a homogeneous mixture was obtained.
Preblend B: 1.00 part glycerine was added to a mixing vessel. 0.1 part Jaguar C17 was added slowly with agitation and the agitation was continued for 15 minutes. 10.0 parts water were added, 20% citric acid solution was added to adjust the pH of the blend to 4.0, and agitation was continued for an additional 15 minutes.
Preblend C: 1.0 part Atlas G-4280 was mixed with 0.25 parts fragrance.
Preblend D: 0.05 parts Dowicil 200 were blended with 0.15 parts water and the mixture was stirred until solution was obtained.

After charging 25.75 parts water to a mixing vessel, 0.19 parts of Polymer JR-400 was added thereto with maintained agitation until a clear solution was obtained. 12.16 parts Tegobetaine L7, 9.50 parts Cedepal TD-403M, 2.85 Monateric 949J and 5.5 parts Atlas G-4280 were added sequentially to the solution with agitation. After Preblend A, which was maintained at a temperature of 65°C, was added with agitation to the solution, Preblend C was then added thereto with agitation. An additional 1.14 parts of Tegobetaine L7 was then added thereto. 0.18 part Versene 100 XL, 3.21 parts of dye solution, preblend D, 4.00 parts Euperlan PK 3000 and 0.75 parts DC 345 were then added sequentially with agitation thereto. Citric acid solution was added in an amount to adjust the pH of the solution to 6.0. Preblend B was then added with agitation. The pH was then checked and adjusted to 6.0 with additional citric acid solution. The amounts of the ingredients used to make the composition of Example 1 are shown in Table 1 below.

The resulting composition was tested for detangling energy and comb force, and the results are provided in Table 2 below: Ocular Irritation of the resulting composition was also measured, and the data are presented in Table 3.

**Table 1**

| **Ingredient** | **INCI Name of Active Ingredient** | **1** | **2** | **1** | **2** |
|---|---|---|---|---|---|
| Atlas G-4280 | PEG-80 Sorbitan Laurate | 6.50 | 6.50 | 6.50 | 6.50 |
| Monateric 949J | Lauroamphoglycinate | 2.85 | 2.85 | 2.85 | 2.85 |
| Tegobetaine L7 | Cocamidopropyl Betaine | 13.30 | 13.30 | 13.30 | 13.30 |
| Cedepal TD-403M | Sodium Trideceth Sulfate | 9.50 | 9.50 | 9.50 | 9.50 |
| Polymer JR 400 | Polyquaternium-10 | 0.19 | 0.19 | 0.19 | --- |
| Jaguar C17 | Guar Hydroxypropyltrimonium Chloride | 0.10 | 0.10 | --- | 0.10 |
| PEG 6000 DS | PEG-150 Distearate | 1.50 | 1.50 | 1.50 | 1.50 |
| Fragrance | | 0.25 | 0.25 | 0.25 | 0.25 |
| Versene 100XL | Tetrasodium EDTA | 0.18 | 0.18 | 0.18 | 0.18 |
| Color (0.10% aq. sol'n) | | 3.21 | 3.21 | 3.21 | 3.21 |
| Dowicil 200 | Quaternium-15 | 0.05 | 0.05 | 0.05 | 0.05 |
| Euperlan PK 3000 | Glycol Distearate, Laureth-4 and Cocamidopropyl Betaine | 4.00 | 4.00 | 4.00 | 4.00 |
| DC 345 Fluid | Cyclomethicone | 0.75 | --- | --- | --- |
| Citric Acid (20% Sol'n) pH to 5.9-6.2 | Citric Acid | 0.85 | 0.85 | 0.85 | 0.85 |
| Glycerin | Glycerin | 1.00 | 1.00 | 1.00 | 1.00 |
| Deionized Water | Deionized Water | Q.S. to 100 | Q.S. to 100 | Q.S. to 100 | Q.S. to 100 |

### Example 2: Compounding of Shampoo Composition without cyclomethicone

The procedure of Example 1 was repeated using the ingredients as set forth in Table 1. The resulting composition was tested for detangling energy and comb force, and the results are provided in Table 2 below:

### Comparative Example 1: Compounding of Shampoo Composition without guar cationic conditioner or cyclomethicone:

The procedure of Example 1 was repeated using the ingredients as set forth in Table 1. The resulting composition was tested for detangling energy and comb force, and the results are provided in Table 2 below:

### Comparative Example 2: Compounding of Shampoo Composition without Polyquaternium-10 or cyclomethicone

The procedure of Example 1 was repeated using the ingredients as set forth in Table 1. The resulting composition was tested for detangling energy and comb force, and the results are provided in Table 2 below:

**Table 2 -**

| **Detangling Energy and Comb Force Measurements** | | | | |
|---|---|---|---|---|
| **Property** | **Example 1** | **Example 2** | **Comparative Example 1** | **Comparative Example 2** |
| Wet Detangling Energy (gram-seconds) | 2218 ± 457 | 2214 ± 683 | 3458 ± 1264 | 3006 ± 630 |
| Wet Comb Force (grams) | 200 ± 25 | 192 ± 51 | 259 ± 77 | 191 ± 58 |
| Dry Detangling Energy (gram-seconds | 2505 ± 1174 | 3162 ± 1386 | 4025 ± 940 | 2891 ± 909 |
| Dry Comb Force (grams) | 169 ± 70 | 164 ± 79 | 161 ± 59 | 146 ± 66 |

As indicated by the data in Table 2, the formulations of Examples 1 and 2, which contain both Polyquatemium-10 and Guar hydroxypropyltrimonium chloride, exhibit significantly improved wet detangling force (lower detangling energy) than either of Comparative examples 1 or 2, each of which contains only one of the conditioners.

In accordance with prior experience, Polyquaternium-10, when used as the sole conditioner in a shampoo formulation, was known to have imparted dry hair managability benefits to the compositions. Similarly, cationic guar compounds have been known to impart wet detangling benefits. Due to the cationic nature of both of these compounds, it was thought that these compounds, when used mixed together, would have competed for the anionic sites on the hair and would thus not have resulted in a shampoo composition exhibiting both improved wet and dry detangling benefits. However, we have unexpectedly found that the combination of cationic cellulose derivatives and cationic guar derivatives in the compositions of this invention imparts superior wet and dry detangling properties to the compositions. More specifically, the wet and dry detangling energy is much lower when using the combination of conditioners of this invention, i.e., cationic guar derivatives and Polyquaternium-10, than if either of the conditioners are used alone.

Example 1 differs from Example 2 in the presence of volatile silicone in formulation. As seen from the data in Table 2, the presence of volatile silicone in Example 1 results in a further reduction in dry detangling energy. Thus, it can be seen that the combination of cationic polymer conditioners and volatile silicone of the compositions of this invention afford both excellent wet detangling and dry detangling benefits.

**Table 3 -**

| **TEP Ocular Irritation Results** | | |
|---|---|---|
| **Formulation** | **Mean EC**_{**50**} | **Rating** |
| Formula of Example 1 | 3.94 ± 1.20 | pass |
| Johnson's Baby Shampoo | 3.34 ± 0.64 | pass |
| Pert Plus for Kids Light Conditioning | 0.74 ± 0.23 | fail |
| Pert Plus for Kids Normal Conditioning | 0.81 ± 0.28 | fail |

As indicated above, formulations which exhibit a mean EC₅₀ value of 2.2 or higher are deemed to pass the ocular irritation test while those exhibiting an EC₅₀ value below 2.2 are deemed to fail the test. As shown in Table 3, the formulation of Example 1 exhibits a passing value, which is on par with Johnson's Baby Shampoo, a commercial shampoo known for its ocular mildness. In contrast, other commercial shampoos, i.e., the Pert Plus products marketed by the Proctor & Gamble Company, fail the test.

Thus, it can be seen from the above Examples that the compositions of the present invention possess superior wet and dry detangling capabilities while retaining low ocular irritation values.

### Examples 3 -15: Preparation of Cleansing Compositions

The following preferred formulations, as shown in Tables 4-7, were made in accordance with the procedure described in Examples 1 and 2.

**Table 4**

| | | **Example** | | |
|---|---|---|---|---|
| **Ingredient** | **INCI Name of Active Ingredient** | **3** | **4** | **5** |
| Atlas G-4280 | PEG-80 Sorbitan Laurate | 6.50 | 6.50 | 6.50 |
| Monateric 949J | Lauroamphoglycinate | 2.85 | 2.85 | 2.85 |
| Tegobetaine L7 | Cocamidopropyl Betaine | 13.30 | 13.30 | 13.30 |
| Cedepal TD-403M | Sodium Trideceth Sulfate | 9.50 | 9.50 | 9.50 |
| Polymer JR 400 | Polyquaternium-10 | 0.14 | --- | 0.10 |
| Jaguar C17 | Guar Hydroxypropyltrimonium Chloride | 0.10 | 0.10 | --- |
| Salcare SC60 | Acrylamidopropyltrimonium Chloride acrylamide copolymer | --- | 0.10 | 0.10 |
| PEG 6000 DS | PEG-150 Distearate | 1.50 | 1.50 | 1.50 |
| Fragrance | | 0.25 | 0.25 | 0.25 |
| Versene 100XL | Tetrasodium EDTA | 0.18 | 0.18 | 0.18 |
| Color (0.10% aq. sol'n) | | 3.21 | 3.21 | 3.21 |
| Dowicil 200 | Quaternium-15 | 0.05 | 0.05 | 0.05 |
| Euperlan PK 3000 | Glycol Distearate, Laureth-4 and Cocamidopropyl Betaine | 4.00 | 4.00 | 4.00 |
| DC 345 Fluid | Cyclomethicone | 0.75 | --- | --- |
| Citric Acid (20% Sol'n) pH to 5.9-6.2 | Citric Acid | 0.85 | 0.85 | 0.85 |
| Glycerin | Glycerin | 1.00 | 1.00 | 1.00 |
| Deionized Water | Deionized Water | Q.S. to 100 | Q.S. to 100 | Q.S. to 100 |

**Table 5**

| | | **Example** | | | |
|---|---|---|---|---|---|
| **Ingredient** | **INCI Name of Active Ingredient** | **6** | **7** | **8** | **9** |
| Tween 20 | Polysorbate 20 | 6.30 | 6.30 | 6.30 | 5.30 |
| Monateric 949-J | Disodium Lauroamphodiacetate | 8.29 | 8.29 | 8.29 | 8.29 |
| Empigen BB/J | Lauryl Betaine | 2.00 | 2.00 | 2.00 | 2.00 |
| Empicol 0251/70-J | Sodium Laureth Sulfate | 4.26 | 4.26 | 4.26 | 4.26 |
| Polymer JR 400 | Polyquaternium-10 | 0.19 | --- | 0.10 | 0.19 |
| Jaguar C17 | Guar hydroxypropyl Trimonium Chloride | 0.10 | 0.10 | --- | 0.10 |
| Salcare SC60 | Acrylamidopropyltrimonium Chloride acrylamide copolymer | --- | 0.12 | 0.10 | --- |
| PEG 6000 DS | PEG-150 Distearate | 1.90 | 1.90 | 1.90 | 1.90 |
| Fragrance | | 0.15 | 0.15 | 0.15 | 0.15 |
| Nervanaid B30 | Tetrasodium EDTA | 0.20 | 0.20 | 0.20 | 0.20 |
| Color 1 (0.2% solution) | | 1.25 | 1.25 | 1.25 | 1.25 |
| Color 2 (0.1% solution) | | 0.30 | 0.30 | 0.30 | 0.30 |
| Genapol 437-X* | Ethylene glycol distearate, cocamidopropyl betaine and cocamide monoethanolamide/diethanolamide | --- | --- | --- | 2.5 |
| Citric Acid (20% Sol'n) | Citric Acid | 1.25 | 1.25 | 1.25 | 1.25 |
| Dowicil 200 | Quaternium-15 | 0.05 | 0.05 | 0.05 | 0.05 |
| Benzyl Alcohol | Benzyl Alcohol | 0.10 | 0.10 | 0.10 | 0.10 |
| Glycerin | Glycerin | 1.00 | 1.00 | 1.00 | 1.00 |
| Deionized Water | Deionized Water | QS to 100 | QS to 100 | QS to 100 | QS to 100 |

| | | | | | |
|---|---|---|---|---|---|
| * Genapol 437-X is a commercial product containing about 20% ethylene glycol distearate, about 6% cocamidopropyl betaine and about 5% cocamide monoethanolamide/diethanolamide available from Hoechst AG, Frankfurt, Germany. | | | | | |

**Table 6**

| | | **Example** | | |
|---|---|---|---|---|
| **Ingredient** | **INCI Name of Active Ingredient** | **10** | **11** | **12** |
| Tween 20 | Polysorbate 20 | 0.50 | 0.50 | 0.50 |
| Empigen BB/J | Lauryl Betaine | 4.20 | 4.20 | 4.20 |
| Ampholak 7CX/C | Sodium Carboxymethyl Cocopolypropylamine | 13.34 | 13.34 | 13.34 |
| Empicol SDD | Disodium Laureth Sulfosuccinate | 13.79 | 13.79 | 13.79 |
| Polymer JR 400 | Polyquaternium-10 | 0.19 | --- | 0.05 |
| Jaguar C17 | Guar hydroxypropyl Trimonium Chloride | 0.10 | 0.10 | --- |
| Salcare SC60 | Acrylamidopropyltrimonium Chloride/acrylamide copolymer | --- | 0.12 | 0.05 |
| PEG 6000 DS (Comiel S.p.A.) | PEG-150 Distearate | 1.95 | 1.95 | 1.95 |
| Fragrance | | 0.14 | 0.14 | 0.14 |
| Versene 100XL | Tetrasodium EDTA | 0.20 | 0.20 | 0.20 |
| Color (0.2% solution) | | 0.23 | 0.23 | 0.23 |
| Citric Acid (20% Sol'n) | Citric Acid | 1.77 | 1.77 | 1.77 |
| Dowicil 200 | Quaternium-15 | 0.05 | 0.05 | 0.05 |
| Glycerin | Glycerin | 1.00 | 1.00 | 1.00 |
| Deionized Water | Deionized Water | QS to 100 | QS to 100 | QS to 100 |

**Table 6**

| | | **Example** | | |
|---|---|---|---|---|
| **Ingredient** | **INCI Name of Active Ingredient** | **10** | **11** | **12** |
| Tween 20 | Polysorbate 20 | 0.50 | 0.50 | 0.50 |
| Empigen BB/J | Lauryl Betaine | 4.20 | 4:20 | 4.20 |
| Ampholak 7CX/C | Sodium Carboxymethyl Cocopolypropylamine | 13.34 | 13.34 | 13.34 |
| Empicol SDD | Disodium Laureth Sulfosuccinate | 13.79 | 13.79 | 13.79 |
| Polymer JR 400 | Polyquaternium-10 | 0.19 | --- | 0.05 |
| Jaguar C17 | Guar hydroxypropyl Trimonium Chloride | 0.10 | 0.10 | --- |
| Salcare SC60 | Acrylamidopropyltrimonium Chloride/acrylamide copolymer | --- | 0.12 | 0.05 |
| PEG 6000 DS (Corniel S.p.A.) | PEG-150 Distearate | 1.95 | 1.95 | 1.95 |
| Fragrance | | 0.14 | 0.14 | 0.14 |
| Versene 100XL | Tetrasodium EDTA | 0.20 | 0.20 | 0.20 |
| Color (0.2% solution) | | 0.23 | 0.23 | 0.23 |
| Citric Acid (20% Sol'n) | Citric Acid | 1.77 | 1.77 | 1.77 |
| Dowicil 200 | Quaternium-15 | 0.05 | 0.05 | 0.05 |
| Glycerin | Glycerin | 1.00 | 1.00 | 1.00 |
| Deionized Water | Deionized Water | QS to 100 | QS to 100 | QS to 100 |

**Table 7**

| | | **Example** | | |
|---|---|---|---|---|
| **Ingredient** | **INCI Name of Active Ingredient** | **13** | **14** | **15** |
| Plantaren 2000N | Decyl Glucoside | 3.60 | 3.60 | 3.60 |
| Atlas G-4280 | PEG-80 Sorbitan Laurate | 3.60 | 3.60 | 3.60 |
| Mirataine CBS | Cocamidopropyl Hydroxysultaine | 6.50 | 6.50 | 6.50 |
| Monateric 1023 | Sodium Lauroampho PG-Acetate Phosphate | 1.45 | 1.45 | 1.45 |
| Cedepal SN-303 | Sodium Laureth Sulfate | 3.60 | 3.60 | 3.60 |
| Polymer JR 400 | Polyquaternium-10 | 0.19 | --- | 0.10 |
| Jaguar C17 | Guar hydroxypropyl Trimonium Chloride | 0.10 | 0.10 | --- |
| Salcare SC60 | Acrylamidopropyltrimonium Chloride acrylamide copolymer | --- | 0.12 | 0.10 |
| Aculyn 22 | Acrylates/Steareth-20 Methacrylate Copolymer | 1.10 | 1.10 | 1.10 |
| PEG 6000 DS | PEG-150 Distearate | 0.72 | 0.72 | 0.72 |
| Fragrance | | | | |
| Versene 100XL | Tetrasodium EDTA | 0.05 | 0.05 | 0.05 |
| Color 1 (0.2% solution) | | | | |
| Color 2 (0.1% solution) | | | | |
| Citric Acid (20% Sol'n) | Citric Acid | 0.46 | 0.46 | 0.46 |
| Sodium Hydroxide (20% Sol'n) | Sodium Hydroxide | 0.32 | 0.32 | 0.32 |
| Dowicil 200 | Quaternium-15 | 0.05 | 0.05 | 0.05 |
| Glycerin | Glycerin | 1.00 | 1.00 | 1.00 |
| Deionized Water | Deionized Water | QS to 100 | QS to 100 | QS to 100 |

Selected compositions from Tables 4 - 7 were evaluated for hair and skin cleansing on human subjects, where the compositions were evaluated for their cleansing, conditioning and irritancy properties. Selected compositions were also evaluated by manual washing and combing of tresses. The compositions were found to be satisfactory conditioning personal cleansing compositions.

### Examples 16 - 54 Preparation of Cleansing Compositions

Additional compositions are prepared in accordance with the procedure set forth in Examples 1 and 2 using the components as set forth in Tables 8-13 below.

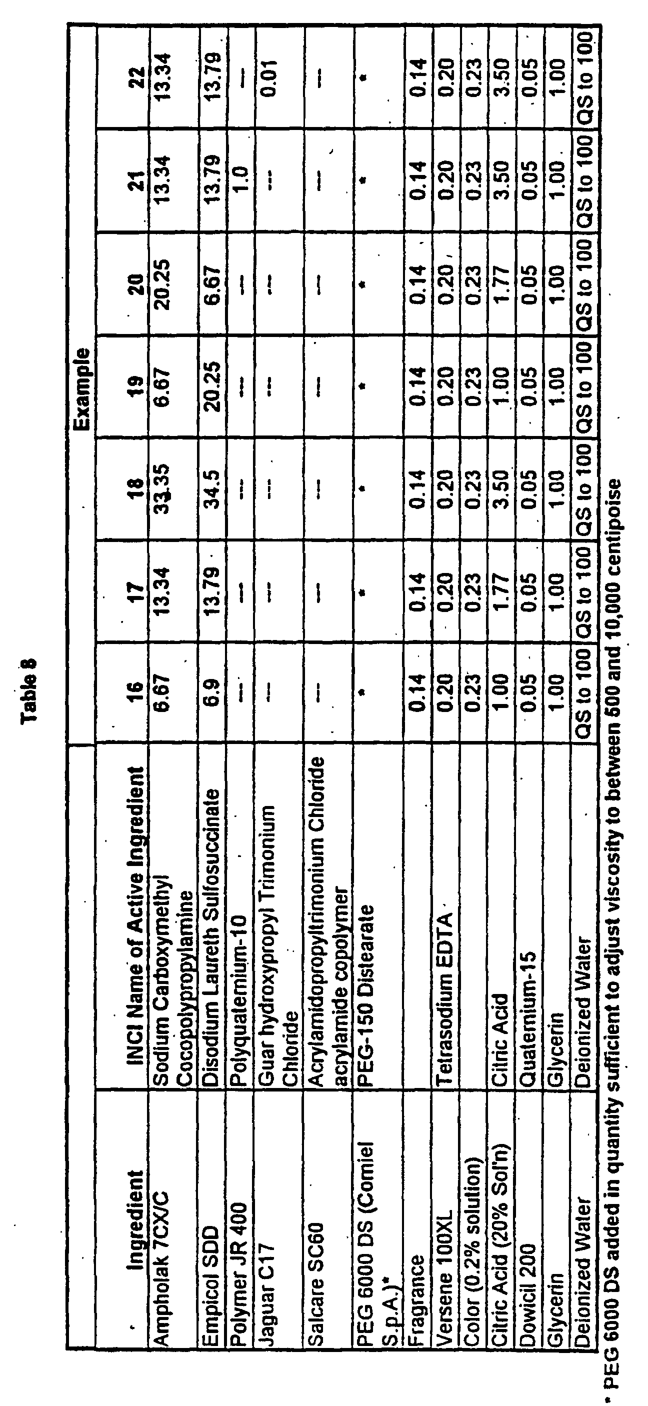

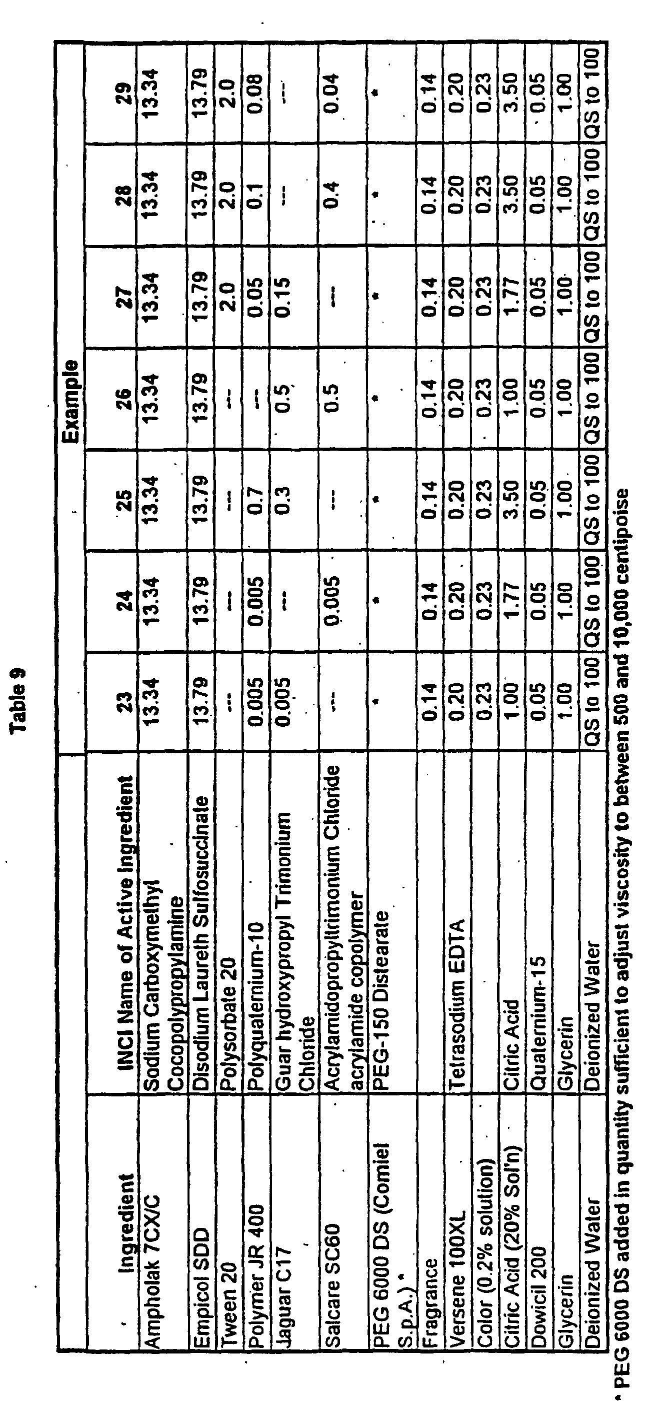

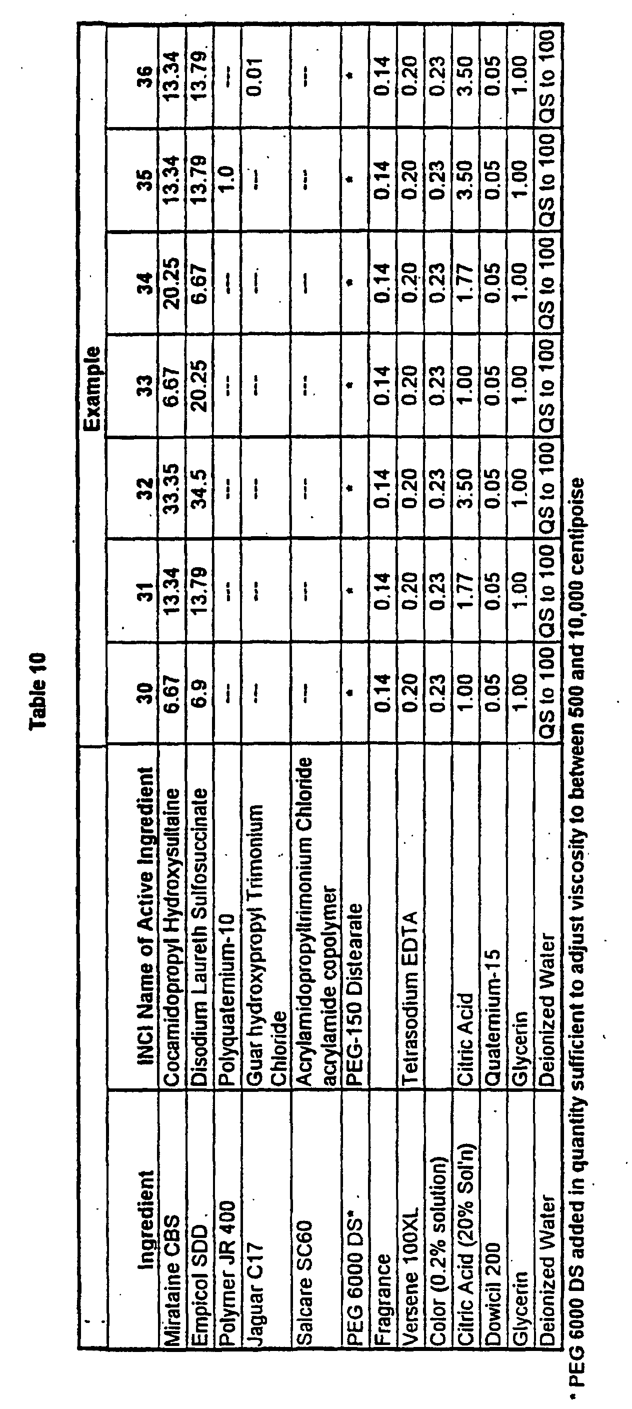

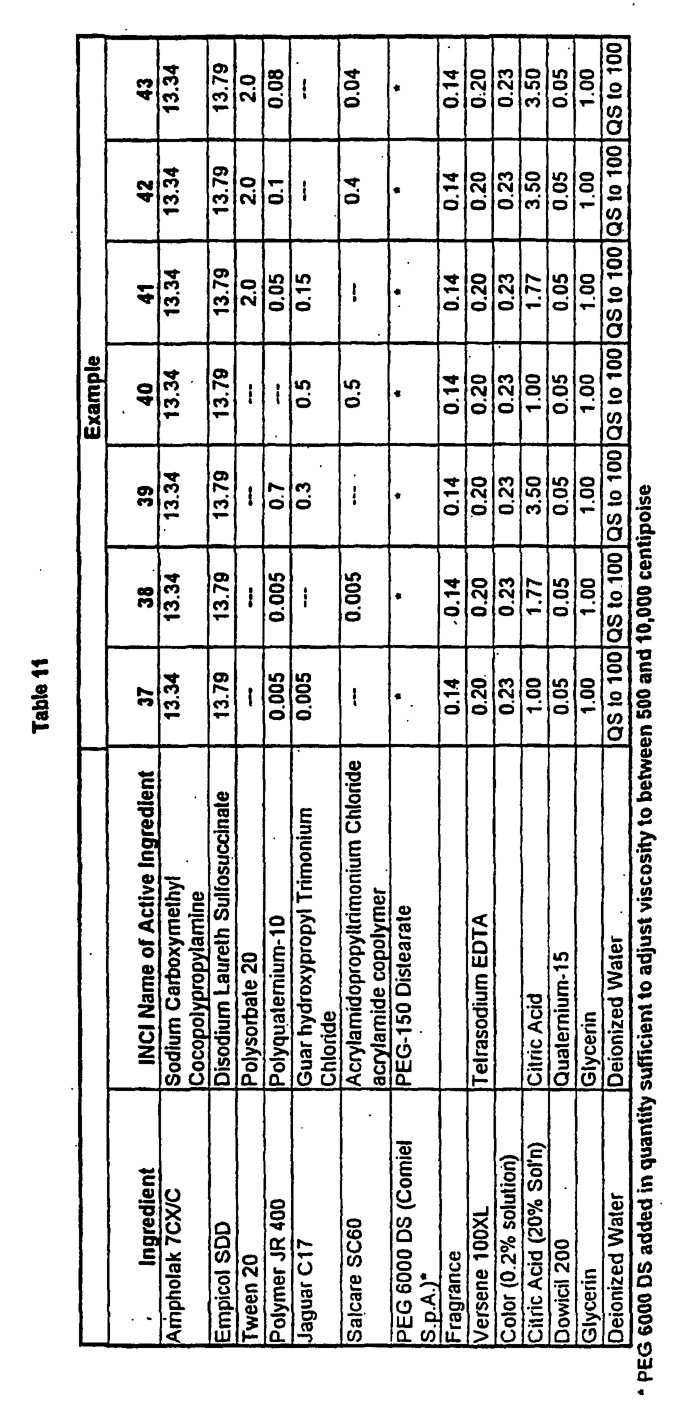

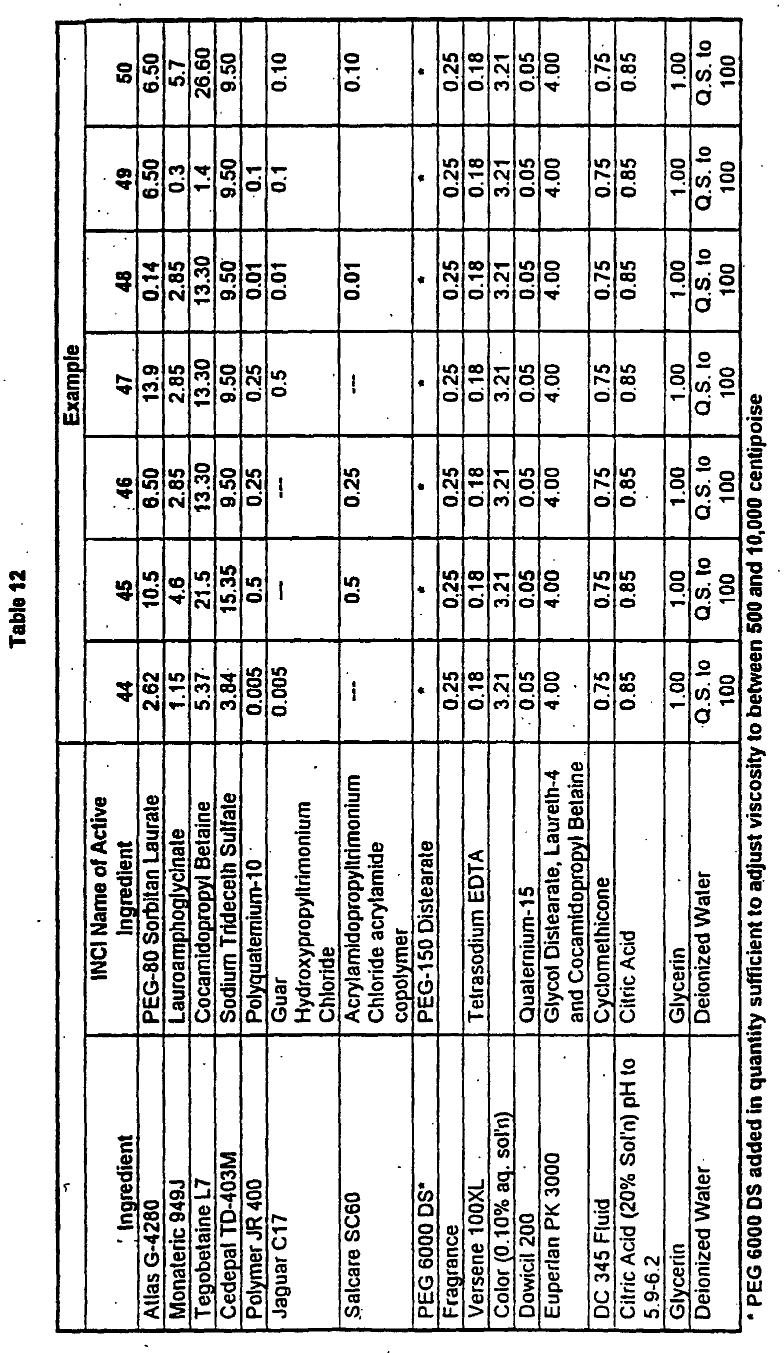

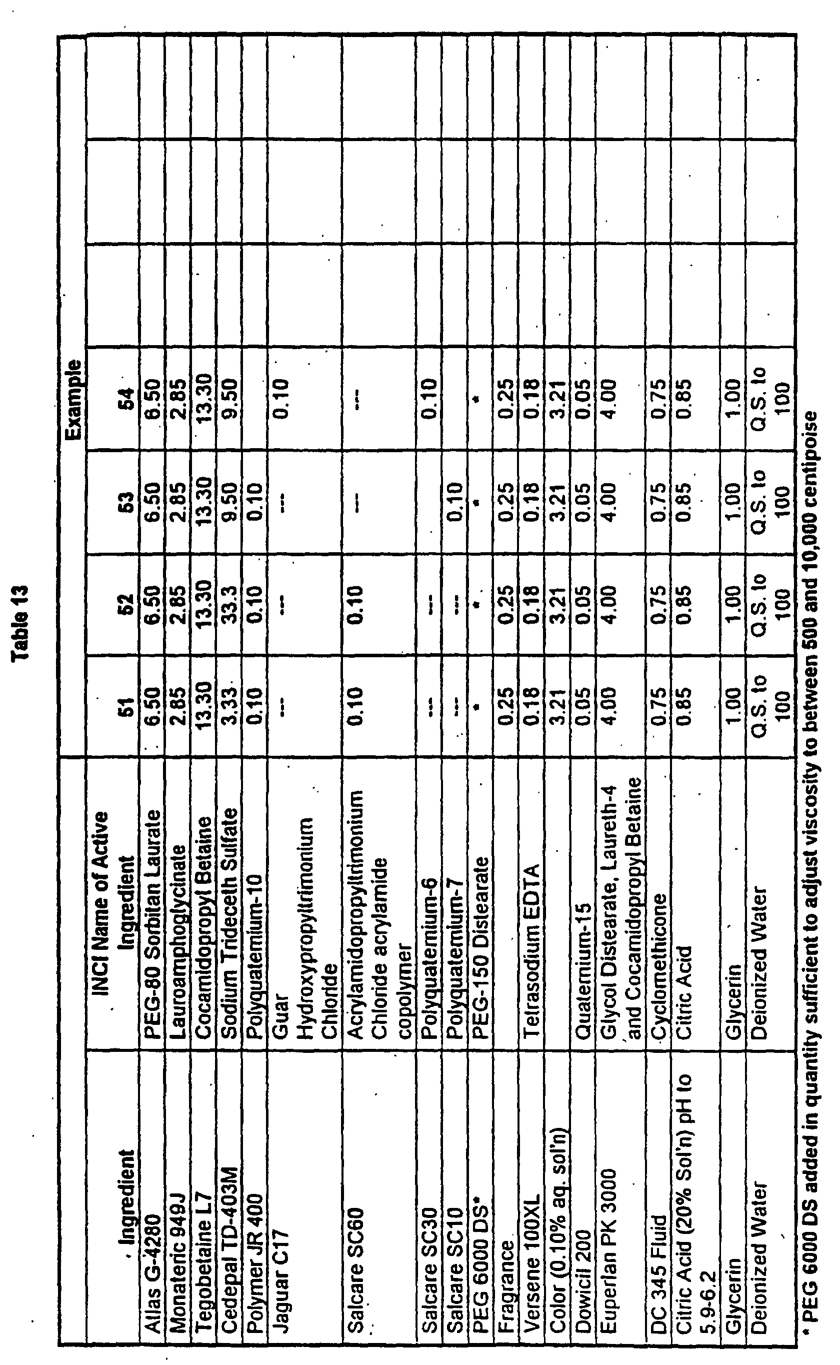

### Example 55: In Vitro Tests

Samples of shampoo compositions shown in Table 14 were evaluated in in vitro tests. In general, members of a test panel were given a blind sample of each respective formulation in Table 14, as well as a blind sample of commercially available Pert Plus for Kids conditioning shampoo ("Pert Plus"). The members were asked to independently use the samples, in approximately equivalents amounts, to shampoo and condition their hair for a given period of time.

The results of the in vitro tests revealed that formulations of Examples 1 and 5 were rated as parity to Pert Plus with respect to ease of conditioning of hair in the dry and wet states. However, the formulation of Example 1 was preferred relative to Pert Plus for the attribute of speed of detangling. By contrast, the formulation of Comparative Example 3, containing only a single cationic polymeric conditioner, was less preferred than Pert Plus with respect to these attributes. These results further illustrated that the formulations of the present invention containing at least two conditioning polymers demonstrated performance superior to shampoos containing only a single conditioning polymer.

**Table 14**

| | | **Example** | | **Comparative Example** |
|---|---|---|---|---|
| **Ingredient** | **INCI Name of Active Ingredient** | **1** | **5** | **3** |
| Atlas G-4280 | PEG-80 Sorbitan Laurate | 6.50 | 6.50 | 6.50 |
| Monateric 949J | Lauroamphoglycinate | 2.85 | 2.85 | 2.85 |
| Tegobetaine L7 | Cocamidopropyl Betaine | 13.30 | 13.30 | 13.30 |
| Cedepal TD-403M | Sodium Trideceth Sulfate | 9.50 | 9.50 | 9.50 |
| Polymer JR 400 | Polyquaternium-10 | 0.19 | 0.10 | --- |
| Jaguar C17 | Guar Hydroxypropyltrimonium Chloride | 0.10 | --- | --- |
| Salcare SC60 | Acrylamidopropyltrimonium chloride acrylamide copolymer | --- | 0.10 | 0.12 |
| PEG 6000 DS | PEG-150 Distearate | 1.50 | 1.50 | 0.50 |
| Fragrance | | 0.25 | 0.25 | 0.25 |
| Versene 100XL | Tetrasodium EDTA | 0.18 | 0.18 | 0.18 |
| Color (0.10% aq. sol'n) | | 3.21 | 3.21 | 3.21 |
| Dowicil 200 | Quaternium-15 | 0.05 | 0.05 | 0.05 |
| Euperlan PK 3000 | Glycol Distearate, Laureth-4 and Cocamidopropyl Betaine | 4.00 | 4.00 | 4.00 |
| DC 345 Fluid | Cyclomethicone | 0.75 | --- | --- |
| Citric Acid (20% Sol'n) pH to 5.9-6.2 | Citric Acid | 0.85 | 0.85 | 0.85 |
| Glycerin | Glycerin | 1.00 | 1.00 | 1.00 |
| Deionized Water | Deionized Water | Q.S. to 100 | Q.S. to 100 | Q.S. to 100 |

## Claims

1. A detergent composition comprising:
a) a surfactant portion comprising:
1. a nonionic surfactant;
2. an amphoteric surfactant; and
3. an anionic surfactant and
b) a conditioner portion comprising at least one cationic conditioning polymer pair selected from:
1. a cationic cellulose derivative and a cationic guar derivative;
2. a cationic guar derivative and a homopolymer or copolymer of a monomer having the formula 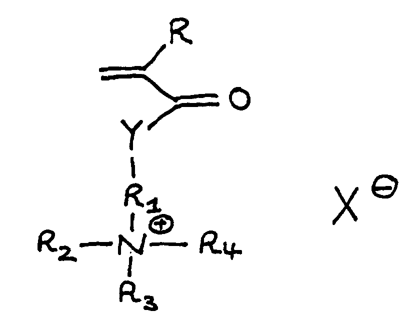 wherein R is H or CH₃, Y is O or NH, R¹ is an alkylene group having from 2 to 6 carbon atoms, R₂, R₃ and R₄ are each independently an alkyl group or hydroxyalkyl group having from 1 to 22 carbon atoms, and X is a monovalent anion selected from halide and alkyl sulfate having from 1 to 4 carbon atoms; or
3. a homopolymer or a copolymer of a cationic monomer of diallyldimethylammonium chloride and a cationic guar derivative.

2. A conditioning detergent composition comprising, based upon the total weight of the detergent composition:
a) from 1 to 10% of nonionic surfactants comprising:
1) a polyoxyethylene derivative of a polyol ester a) derived from a fatty acid containing from 8 to 22 carbon atoms and a polyol selected from sorbitol, sorbitan, glucose., 1-methyl glucoside, polyglucose having an average of 1 to 3 glucose residues, glycerine, pentaerythritol and mixtures thereof, b) containing an average of from 10 to 120 oxyethylene units, and c) having an average of from 1 to 3 fatty acid residues per molecule of the polyoxyethylene derivative of polyol ester,
2) an alkyl glucoside having an alkyl group containing from 6 to 22 carbon atoms and having from 1 to 6 glucose residues per molecule of alkyl glucoside, or
3) mixtures thereof, and
b) from 0.5 to 10% of one or more amphocarboxylate amphoteric surfactants of the formula A-CONH(CH₂)ₓN⁺R₅R₆R₇ wherein: A is an alkyl or alkenyl group having from 7 to 21 carbon atoms; x is an integer of from 2 to 6; R₅ is hydrogen or a carboxyalkyl group containing from 2 to 3 carbon atoms; R₆ is a hydroxyalkyl group containing from 2 to 3 carbon atoms or a group of the formula R₈-O- (CH₂)ₙCO₂⁻ wherein R₈ is an alkylene group having from 2 to 3 carbon atoms and n is 1 or 2; and R₇ is a carboxyalkyl group containing from 2 to 3 carbon atoms,
c. from 0.5 to 10% of one or more betaine amphoteric surfactants selected from:
1) an alkyl betaine of the formula B-N⁺R₉R₁₀(CH₂) ₚCO₂⁻ wherein: B is an alkyl or alkenyl group having from 8 to 22 carbon atoms; R₉ and R₁₀ are each independently an alkyl group or a hydroxyalkyl group having from 1 to 4 carbon atoms; and p is 1 or 2; or
2) an amidoalkyl betaine of the formula
D-CO-NH (CH₂)_{q}-N⁺R₁₁R₁₂ (CH₂)ₘ CO₂⁻ wherein D is an alkyl or alkenyl group having from 7 to 21 carbon atoms; R₁₁ and R₁₂ are each independently an alkyl group or a hydroxyalkyl group having from 1 to 4 carbon atoms; q is an integer from 2 to 6; and m is 1 or 2;
d. from 1 to 10% of one or more anionic alkyl ether sulfate surfactants of the formula R'(OCH₂CH₂)ᵥOSO₃X', wherein R' is an alkyl or alkenyl group having from 7 to 22 carbon atoms, X' is an alkali metal ion, alkaline earth metal ion, ammonium ion, ammonium ion substituted with from 1 to 3 substituents, each of which substituents may be the same or different and is an alkyl group having from 1 to 4 carbon atoms or a hydroxyalkyl group having from 2 to 4 carbon atoms, and v is an integer from 1 to 6;
e. from 0.01 to 0.5% of Polyquaternium-10; and
f. from 0.01 to 0.5% of guar hydroxypropyltrimonium chloride;
wherein the surfactants in a through d are present in an amount, based upon the total weight of the detergent composition, from 5 percent to 20 percent.

3. The detergent composition of claim 2 further comprising from 0.25 to 2.5%, based upon the total weight of the detergent composition, of a pearlescent or opacifying agent comprising a diester of : a) a fatty acid having from 16 to 22 carbon atoms; and b) ethylene glycol or propylene glycol.

4. The detergent composition of claim 3 wherein the pearlescent or opacifying agent is added to the detergent composition as a preformed, stabilized aqueous dispersion.

5. The detergent composition of any one of claims 2 to 4 further comprising from 0.5 to 5%, based upon the total weight of the detergent composition, of a humectant comprising glycerine.

6. The detergent composition of any one of claims 2 to 5 further comprising a thickener comprising a diester of: a) polyethylene glycol of formula
HO-(CH₂CH₂O)z-H wherein z is an integer from 3 to 200; and b) a fatty acid containing from 16 to 22 carbon atoms, wherein the thickener is present in an amount sufficient to provide the detergent composition with a Brookfield viscosity between 500 centipoise to 10,000 centipoise.

7. The detergent composition of any one of claims 2 to 6 further comprising, based upon the total weight of the detergent composition, from 0.25 to 2.5% of a silicone-based conditioner comprised of polydimethylsiloxane, polydimethylcyclosiloxane, hexamethyldisiloxane, or a mixture thereof.

8. The detergent composition of claim 7 wherein the silicone-based conditioner comprises a volatile silicone compound having an atmospheric pressure boiling point of less than about 220°C.

9. The detergent composition of any one of claims 2 to 8 further comprising additives comprised of colorants, fragrances, preservatives, pH adjusting agents, and mixtures thereof.

10. The detergent composition of any one of claims 2 to 9 wherein the detergent composition has a pH of from 5 to 7.5.

11. A conditioning detergent composition comprising, based upon the total weight of the detergent composition:
A. from 5 to 20% of a surfactant portion comprising, based upon the total weight of the detergent composition,
1. 2.0 to 8% of a nonionic surfactant comprising a polyoxyethylene derivative of polyol ester, an alkyl glucoside wherein the alkyl group contains from 8 to 14 carbon atoms, or a mixture thereof;
2. 1 to 6% of an amidoalkyl sultaine amphoteric surfactant; and
3. from 1 to 6% of an alkyl ether sulfate anionic surfactant; and
B. from 0.01 to 1.0% of a conditioner portion comprising at least one cationic conditioning polymer pair selected from:
1. a cationic cellulose derivative and a cationic guar derivative;
2. a cationic guar derivative and a homopolymer or copolymer of a monomer having a formula 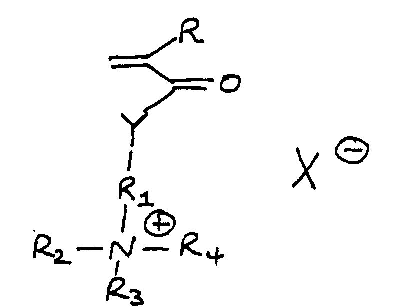 wherein R is H or CH₃, Y is O or NH, R₁ is an alkylene group having from 2 to 6 carbon atoms, R₂, R₃ and R₄ are each independently an alkyl group or hydroxyalkyl group having from 1 to 22 carbon atoms, and X is a monovalent anion selected from halide and alkyl sulfate having from 1 to 4 carbon atoms; or
3. a homopolymer or a copolymer of a cationic monomer of diallyldimethylammonium chloride and a cationic guar derivative

12. A conditioning detergent composition comprising, based upon the total weight of the detergent composition:
A. from 5 to 20% of a surfactant portion comprising, based upon the total weight of the detergent composition:
1. from 0.1 to 8% of a nonionic surfactant comprised of
a) a polyoxyethylene derivative of polyol ester;
b) an alkyl glucoside, the alkyl group having from 8 to 14 carbon atoms; or
c) mixtures thereof;
2. from 2 to 6% of a carboxyalkyl alkyl polyamine amphoteric surfactant of the formula: 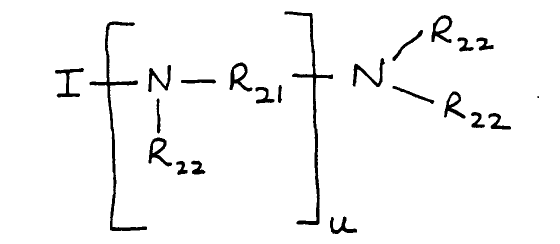 wherein I is an alkyl or alkenyl group containing from 8 to 22 carbon atoms; R₂₂ is a carboxyalkyl group having from 2 to 3 carbon atoms; R₂₁ is an alkylene group having from 2 to 3 carbon atoms; and u is an integer from 2 to 4;
3. from 2 to 8% of an anionic surfactant which is an alkyl ether sulfosuccinate, alkyl ether sulfate, or a mixture thereof, the alkyl group having from 8 to 14 carbon atoms; and
B. 0.01 to 1.0% of a conditioner portion comprising at least two cationic conditioning polymers selected from:
1. a cationic cellulose derivative;
2. a cationic guar derivative
3. a homopolymer or copolymer of a cationic monomer selected from:
a. a monomer having the formula 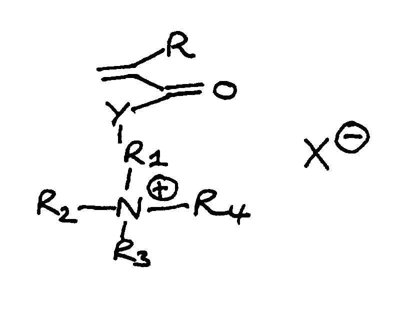 wherein R is H or CH₃, Y is O or NH, R₁ is an alkylene group having from 2 to 6 carbon atoms, R₂, R₃ and R₄ are each independently an alkyl group or a hydroxyalkyl group having from 1 to 22 carbon atoms, and X is a monovalent anion selected from halide and alkyl sulfate having from 1 to 4 carbon atoms, or
b. diallyldimethylammonium chloride.

13. A detergent composition comprising:
a. sodium carboxymethyl cocopolypropylamine as an amphoteric surfactant;
b. an anionic surfactant, except those anionic surfactants of the group consisting of
(i) an alkyl sulfate of the formula R'-CH₂OSO₃X', and
(ii) an alkylaryl sulfonate of the formula 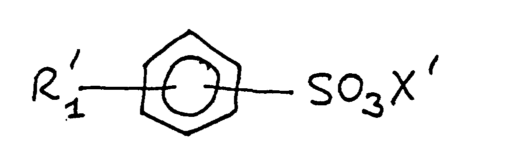 wherein R' is an alkyl group having from 7 to 14 carbon atoms, R'₁ is an alkyl group having from 1 to 12 carbon atoms and X' is an alkali metal ion, alkaline earth metal ion, ammonium ion or ammonium ion substituted with from 1 to 3 substituents; each of which substituents may be the same or different and is an alkyl group having 1 to 4 carbon atoms or a hydroxyalkyl group having from 2 to 4 carbon atoms;
and
c. optionally a non-ionic surfactant,
with the proviso that if the non-ionic surfactant is omitted and the anionic surfactant is an alkyl ether sulfate of the formula R'(OCH₂CH₂)ᵥOSO₃X', then v is greater than or equal to 3.

14. The detergent composition of claim 13 wherein the anionic surfactant is an alkyl ether sulfosuccinate of the formula: 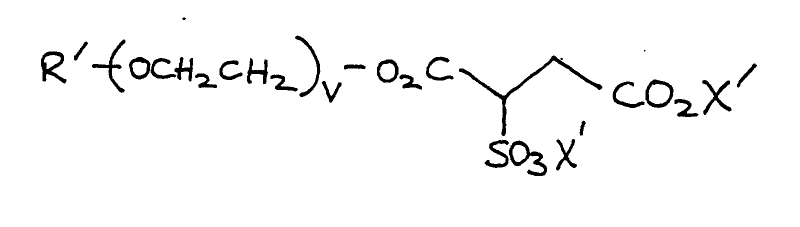 wherein R' is an alkyl group having from 7 to 22 carbon atoms, X' is an alkali metal ion, alkaline earth metal ion, ammonium ion, or an ammonium ion substituted with from 1 to 3 substituents, each of which substituents may be the same or different and is an alkyl group having from 1 to 4 carbon atoms or a hydroxyalkyl group having from 2 to 4 carbon atoms and v is an integer from 1 to 6
or mixtures thereof.

15. The detergent composition of claim 13 or claim 14 further comprising at least one conditioner selected from:
1. a cationic cellulose derivative;
2. a cationic guar derivative; and
3. a homopolymer or copolymer of a cationic monomer selected from:
a. a monomer having the formula 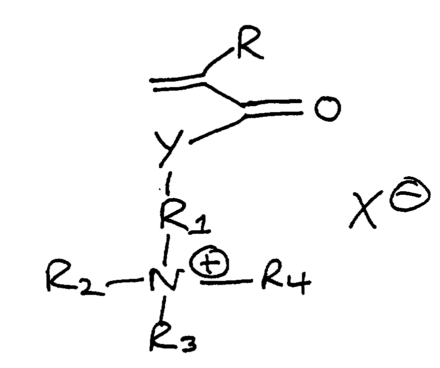 wherein R is H or CH₃, Y is O or NH, R₁ is an alkylene group having from 2 to 6 carbon atoms, R₂, R₃ and R₄ are each independently an alkyl group or hydroxyalkyl group having from 1 to 22 carbon atoms, and X is a monovalent anion selected from halide and alkyl sulfate having from 1 to 4 carbon atoms, or
b. diallyldimethylammonium chloride.

16. A detergent composition comprising:
a. sodium carboxymethyl cocopolypropylamine as an amphoteric surfactant;
b. an anionic surfactant;
c. optionally a non-ionic surfactant; and
d. at least two conditioners selected from:
1. a cationic cellulose derivative;
2. a cationic guar derivative; and
3. a homopolymer or copolymer of a cationic monomer selected from:
a. a monomer having the formula 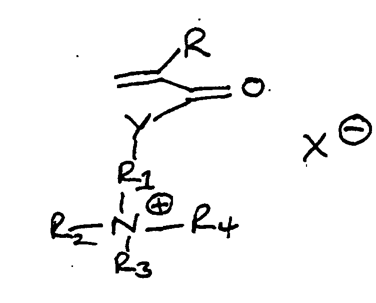 wherein R is H or CH₃, Y is O or NH, R₁ is an alkylene group having from 2 to 6 carbon atoms, R₂, R₃ and R₄ are each independently an alkyl group or alkyl modified with a hydroxy group having from 1 to 22 carbon atoms, and X is a monovalent anion selected from halide and alkyl sulfate having from 1 to 4 carbon atoms, or
b. diallyldimethylammonium chloride.

17. A detergent composition comprising
a. an amidoalkyl sultaine amphoteric surfactant of the formula: 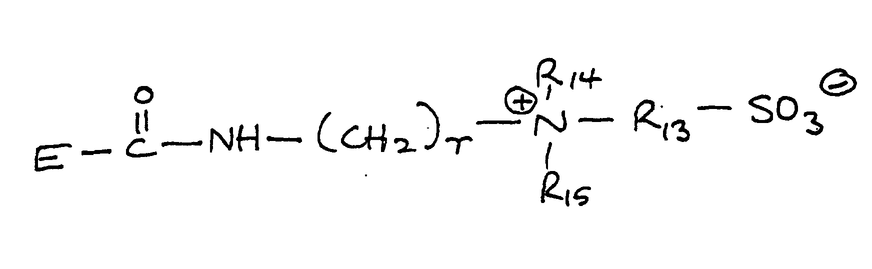 wherein E is an alkyl group or alkenyl group having from 7 to 21 carbon atoms; R₁₄ and R₁₅ are each independently an alkyl group or a hydroxyalkyl group having from 1 to 4 carbon atoms; r is an integer from 2 to 6; and R₁₃ is an alkylene or hydroxyalkylene group having from 2 to 3 atoms;
b. an ionic surfactant,
c. optionally a non-ionic surfactant, and
d. at least one conditioner pair selected from:
1. a cationic cellulose derivative and a cationic guar derivative;
2. a cationic guar derivative and a homopolymer or copolymer of diallyldimethylammonism chloride;
3. a homopolymer or copolymer of a monomer having the formula 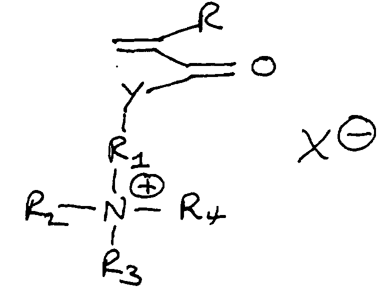 wherein R is H or CH₃, Y is O or NH, R₁ is an alkylene group having from 2 to 6 carbon atoms, R₂, R₃ and R₄ are each independently an alkyl group or hydroxyalkyl group having from 1 to 22 carbon atoms, and X is a monovalent anion selected from halide and alkyl sulfate having from 1 to 4 carbon atoms
and a cationic guar derivative.

## Patentansprüche

1. Detergens-Zusammensetzung, die folgendes umfaßt:
a) einen Anteil grenzflächenaktives Agens, umfassend:
1. ein nicht-ionisches grenzflächenaktives Agens;
2. ein amphoteres grenzflächenaktives Agens; und
3. ein anionisches grenzflächenaktives Agens; und
b) einen Anteil Konditionierer, umfassend mindestens ein kationisches konditionierendes Polymer-Paar, ausgewählt aus:
1. einem kationischen Zellulose-Derivat und einem kationischen Guar-Derivat;
2. einem kationischen Guar-Derivat und einem Homopolymer oder Co-Polymer eines Monomers mit der Formel: 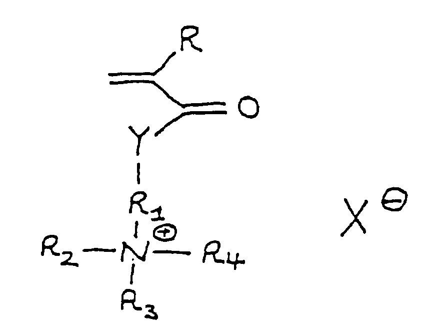 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder Hydroxyalkyl-Gruppe mit 1 bis 22 Kohlenstoffatomen sind; und X ein monovalentes Anion, ausgewählt aus Halogenid und Alkylsulfat mit 1 bis 4 Kohlenstoffatomen, ist; oder
3. einem Homopolymer oder einem Co-Polymer eines kationischen Monomers von Diallyldimethylammoniumchlorid und einem kationischen Guar-Derivat.

2. Eine konditionierende Detergens-Zusammensetzung, die, basierend auf dem Gesamtgewicht der Detergens-Zusammensetzung, folgendes umfaßt:
a) 1 bis 10% nicht-ionische grenzflächenaktive Agenzien, umfassend:
1. ein Polyoxyethylen-Derivat eines Polyolesters, a) abgeleitet von einer Fettsäure, die 8 bis 22 Kohlenstoffatome enthält, und einem Polyol, ausgewählt aus Sorbit, Sorbitan, Glukose, 1-Methylglukosid, Polyglukose mit durchschnittlich 1 bis 3 Glukose-Resten, Glycerin, Pentaerythrit und Mischungen davon, b) im Durchschnitt 10 bis 120 Oxyethylen-Einheiten enthaltend, und c) im Durchschnitt 1 bis 3 FettsäureReste pro Molekül des Polyoxyethylen-Derivats von Polyolester aufweisend;
2. ein Alkylglukosid mit einer Alkyl-Gruppe, die 6 bis 22 Kohlenstoffatome enthält, und das 1 bis 6 Glukose-Reste pro Molekül des Alkylglukosids aufweist; oder
3. Mischungen davon; und
b) 0,5 bis 10% eines oder mehrerer amphoterer grenzflächenaktiver Amphocarboxylsäure-Agenzien der Formel A-CONH(CH₂)ₓN⁺R₅R₆R₇, wobei A eine Alkyl- oder Alkenyl-Gruppe mit 7 bis 21 Kohlenstoffatomen ist; x eine ganze Zahl von 2 bis 6 ist; R₅ Wasserstoff oder eine Carboxyalkyl-Gruppe, die 2 bis 3 Kohlenstoffatome enthält, ist; R₆ eine Hydroxyalkyl-Gruppe, die 2 bis 3 Kohlenstoffatome enthält, oder eine Gruppe der Formel R₈-O-(CH₂)ₙCO₂⁻ ist, wobei R₈ eine Alkylen-Gruppe mit 2 bis 3 Kohlenstoffatomen ist, und n 1 oder 2 ist; und R₇ eine Carboxyalkyl-Gruppe, die 2 bis 3 Kohlenstoffatomen enthält, ist;
c) 0,5 bis 10% eines oder mehrerer amphoterer oberflächenaktiver Betain-Agenzien, ausgewählt aus:
1. einem Alkyl-Betain der Formel B-N⁺R₉R₁₀(CH₂)ₚCO₂⁻, wobei B eine Alkyl- oder Alkenyl-Gruppe mit 8 bis 22 Kohlenstoffatomen ist; R₉ und R₁₀ jeweils unabhängig eine Alkyl-Gruppe oder eine Hydroxyalkyl-Gruppe mit 1 bis 4 Kohlenstoffatomen sind; und p 1 oder 2 ist; oder
2. einem Amidoalkyl-Betain der Formel
D-CO-NH (CH₂)_{q}-N⁺R₁₁R₁₂(CH₂)ₘCO₂⁻, wobei D eine Alkyl- oder Alkenyl-Gruppe mit 7 bis 21 Kohlenstoffatomen ist; R₁₁ und R₁₂ jeweils unabhängig eine Alkyl-Gruppe oder eine Hydroxyalkyl-Gruppe mit 1 bis 4 Kohlenstoffatomen sind; q eine ganze Zahl von 2 bis 6 ist; und m 1 oder 2 ist;
d) 1 bis 10% eines oder mehrerer anionischer grenzflächenaktiver Alkylethersulfat-Agenzien der Formel R'(OCH₂CH₂)ᵥOSO₃X', wobei R' eine Alkyl- oder Alkenyl-Gruppe mit 7 bis 22 Kohlenstoffatomen ist; X' ist ein Alkalimetall-Ion, Erdalkalimetall-Ion, Ammonium-Ion, Ammonium-Ion, das durch 1 bis 3 Substituenten substituiert ist, wobei jeder der Substituenten gleich oder verschieden sein kann und eine Alkyl-Gruppe mit 1 bis 4 Kohlenstoffatomen oder eine Hydroxyalkyl-Gruppe mit 2 bis 4 Kohlenstoffatomen ist; und v eine ganze Zahl von 1 bis 6 ist;
e) 0,01 bis 0,5% Polyquaternium-10; und
f) 0,01 bis 0,5% Guar-Hydroxypropyltriammoniumchlorid;
wobei die grenzflächenaktiven Agenzien in a bis d in einer Menge von 5 Prozent bis 20 Prozent, basierend auf dem Gesamtgewicht der Detergens-Zusammensetzung, vorhanden sind.

3. Detergens-Zusammensetzung nach Anspruch 2, weiterhin umfassend 0,25 bis 2,5%, basierend auf dem Gesamtgewicht der Detergens-Zusammensetzung, eines irisierenden oder Opaleszenz-verleihenden Agens, das einen Diester aus: a) einer Fettsäure mit 16 bis 22 Kohlenstoffatomen; und b) Ethylenglykol oder Propylenglykol umfaßt.

4. Detergens-Zusammensetzung nach Anspruch 3, wobei das irisierende oder Opaleszenz-verleihende Agens der Detergens-Zusammensetzung als eine vorgefertigte, stabilisierte, wäßrige Dispersion zugesetzt wird.

5. Detergens-Zusammensetzung nach einem der Ansprüche 2 bis 4, weiterhin umfassend 0,5 bis 5%, basierend auf dem Gesamtgewicht der Detergens-Zusammensetzung, eines Befeuchtungsmittels, das Glycerin umfaßt.

6. Detergens-Zusammensetzung nach einem der Ansprüche 2 bis 5, weiterhin umfassend einen Verdicker, der einen Diester umfaßt aus: a) Polyethylenglykol der Formel HO-(CH₂CH₂O)_{z}-H, wobei z eine ganze Zahl von 3 bis 200 ist; und b) einer Fettsäure, die 16 bis 22 Kohlenstoffatome enthält, wobei der Verdicker in einer Menge vorhanden ist, die ausreicht, der Detergens-Zusammensetzung eine Brookfield-Viskosität zwischen 500 centipoise bis 10.000 centipoise zu verleihen.

7. Detergens-Zusammensetzung nach einem der Ansprüche 2 bis 6, die weiterhin 0,25 bis 2,5%, basierend auf dem Gesamtgewicht der Detergens-Zusammensetzung, eines Konditionierers auf der Basis von Silikon, bestehend aus Polydimethylsiloxan, Polydimethylcyclosiloxan, Hexamethyldisiloxan oder einer Mischung daraus, umfaßt.

8. Detergens-Zusammensetzung nach Anspruch 7, wobei der Konditionierer auf Silikon-Basis eine volatile Silikon-Verbindung mit einem Siedepunkt bei atmosphärischem Druck von weniger als ungefähr 220°C umfaßt.

9. Detergens-Zusammensetzung nach einem der Ansprüche 2 bis 8, die weiterhin Additive, bestehend aus Farbstoffen, Duftstoffen, Konservierungsstoffen, pH-adjustierenden Agenzien und Mischungen davon, umfaßt.

10. Detergens-Zusammensetzung nach einem der Ansprüche 2 bis 9, wobei die Detergens-Zusammensetzung ein pH von 5 bis 7,5 aufweist.

11. Eine konditionierende Detergens-Zusammensetzung, die, basierend auf dem Gesamtgewicht der Detergens-Zusammensetzung, folgendes umfaßt:
A. 5 bis 20%, basierend auf dem Gesamtgewicht der Detergens-Zusammensetzung, eines Anteils grenzflächenaktives Agens, umfassend:
1. 2,0 bis 8% eines nicht-ionischen grenzflächenaktiven Agens, umfassend ein Polyoxyethylen-Derivat von Polyolester, ein Alkylglukosid, wobei die Alkyl-Gruppe 8 bis 14 Kohlenstoffatome enthält, oder ein Gemisch davon;
2. 1 bis 6% eines amphoteren grenzflächenaktiven Amidoalkylsultain-Agens; und
3. 1 bis 6% eines anionischen grenzflächenaktiven Alkylether-Agens; und
B. 0,01 bis 1,0% eines Anteils Konditionierer, umfassend mindestens ein kationisches konditionierendes Polymer-Paar, ausgewählt aus:
1. einem kationischen Zellulose-Derivat und einem kationischen Guar-Derivat;
2. einem kationischen Guar-Derivat und einem Homopolymer oder Co-Polymer eines Monomers mit einer Formel: 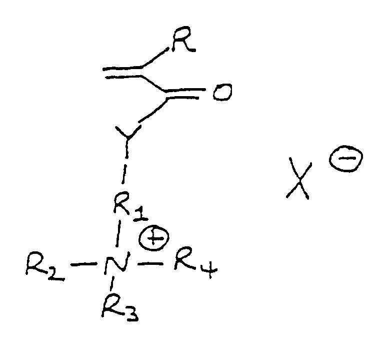 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder Hydroxyalkyl-Gruppe mit 1 bis 22 Kohlenstoffatomen sind; und X ein monovalentes Anion, ausgewählt aus Halogenid und Alkylsulfat mit 1 bis 4 Kohlenstoffatomen, ist; oder
3. einem Homopolymer oder einem Co-Polymer eines kationischen Monomers von Diallyldimethylammoniumchlorid und einem kationischen Guar-Derivat.

12. Eine konditionierende Detergens-Zusammensetzung, die, basierend auf dem Gesamtgewicht der Detergens-Zusammensetzung, folgendes umfaßt:
A. 5 bis 20%, basierend auf dem Gesamtgewicht der Detergens-Zusammensetzung, eines Anteils grenzflächenaktives Agens, umfassend:
1. 0,1 bis 8% eines nicht-ionischen grenzflächenaktiven Agens, bestehend aus:
a) einem Polyoxyethylen-Derivat von Polyolester;
b) einem Alkylglukosid, wobei die Alkyl-Gruppe 8 bis 14 Kohlenstoffatome aufweist; oder
c) Mischungen davon;
2. 2 bis 6% eines amphoteren grenzflächenaktiven Carboxyalkylalkylpolyamin-Agens der Formel: 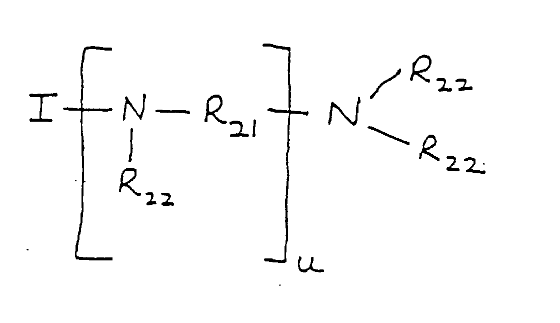 wobei I eine Alkyl- oder Alkylen-Gruppe ist, die 8 bis 22 Kohlenstoffatome enthält; R₂₂ eine Carboxyalkyl-Gruppe mit 2 bis 3 Kohlenstoffatomen ist; R₂₁ eine Alkylen-Gruppe mit 2 bis 3 Kohlenstoffatomen ist; und u eine ganze Zahl von 2 bis 4 ist;
3. 2 bis 8% eines anionischen grenzflächenaktiven Agens, das ein Alkylethersulfosuccinat, Alkylethersulfat oder ein Gemisch davon ist, wobei die Alkyl-Gruppe 8 bis 14 Kohlenstoffatome aufweist; und
B. 0,01 bis 1,0% eines Anteils Konditionierer, der mindestens zwei kationische konditionierende Polymere umfaßt, ausgewählt aus:
1. einem kationischen Zellulose-Derivat;
2. einem kationischen Guar-Derivat;
3. einem Homopolymer oder Co-Polymer eines kationischen Monomers, ausgewählt aus:
a. einem Monomer mit der Formel: 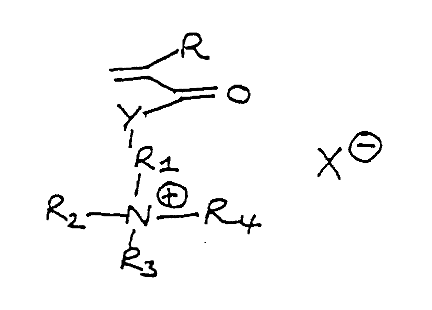 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder eine Hydroxyalkyl-Gruppe mit 1 bis 22 Kohlenstoffatomen sind; und X ein monovalentes Anion, ausgewählt aus Halogenid und Alkylsulfat mit 1 bis 4 Kohlenstoffatomen, ist; oder
b. Diallyldimethylammoniumchlorid.

13. Detergens-Zusammensetzung, die folgendes umfaßt:
a. Natriumcarboxymethylcocopolypropylamin als ein amphoteres grenzflächenaktives Agens;
b. ein anionisches grenzflächenaktives Agens, außer jenen anionischen grenzflächenaktiven Agenzien der Gruppe, die besteht aus:
(i) einem Alkylsulfat der Formel R'-CH₂OSO₃X'; und
(ii) einem Alkylarylsulfonat der Formel: 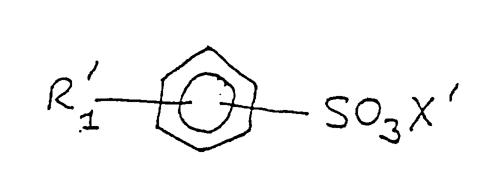
wobei R' eine Alkyl-Gruppe mit 7 bis 14 Kohlenstoffatomen ist; R'₁ eine Alkyl-Gruppe mit 1 bis 12 Kohlenstoffatomen ist; und X' ist ein Alkalimetall-Ion, Erdalkalimetall-Ion, Ammonium-Ion oder Ammonium-Ion, das durch 1 bis 3 Substituenten substituiert ist, wobei jeder der Substituenten gleich oder verschieden sein kann und eine Alkyl-Gruppe mit 1 bis 4 Kohlenstoffatomen oder eine Hydroxyalkyl-Gruppe mit 2 bis 4 Kohlenstoffatomen ist; und
c. wahlweise ein nicht-ionisches grenzflächenaktives Agens, unter der Bedingung, daß, wenn auf das nicht-ionische grenzflächenaktive Agens verzichtet wird und das anionische grenzflächenaktive Agens ein Alkylethersulfat der Formel R'(OCH₂CH₂)ᵥOSO₃X' ist, v dann größer oder gleich 3 ist.

14. Detergens-Zusammensetzung nach Anspruch 13, wobei das anionische grenzflächenaktive Agens ein Alkylethersulfosuccinat der folgenden Formel ist: 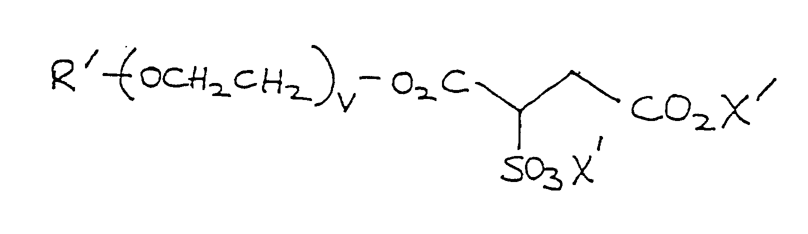 wobei R' eine Alkyl-Gruppe mit 7 bis 22 Kohlenstoffatomen ist; X' ist ein Alkalimetall-Ion, Erdalkalimetall-Ion, Ammonium-Ion oder ein Ammonium-Ion, das durch 1 bis 3 Substituenten substituiert ist, wobei jeder der Substituenten gleich oder verschieden sein kann und eine Alkyl-Gruppe mit 1 bis 4 Kohlenstoffatomen oder eine Hydroxyalkyl-Gruppe mit 2 bis 4 Kohlenstoffatomen ist; und v eine ganze Zahl von 1 bis 6 ist;
oder Mischungen davon.

15. Detergens-Zusammensetzung nach Anspruch 13 oder Anspruch 14, die weiterhin mindestens einen Konditionierer umfaßt, ausgewählt aus:
1. einem kationischen Zellulose-Derivat;
2. einem kationischen Guar-Derivat; und
3. einem Homopolymer oder Co-Polymer eines kationischen Monomers, ausgewählt aus:
a. einem Monomer mit der Formel: 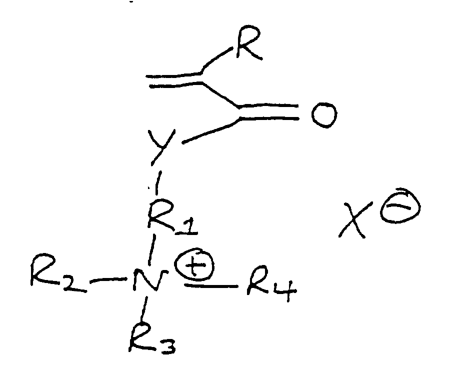 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder Hydroxyalkyl-Gruppe mit 1 bis 22 Kohlenstoffatomen sind; und X ein monovalentes Anion, ausgewählt aus Halogenid und Alkylsulfat mit 1 bis 4 Kohlenstoffatomen, ist; oder
b. Diallyldimethylammoniumchlorid.

16. Eine Detergens-Zusammensetzung, die folgendes umfaßt:
a. Natriumcarboxymethylcocopolypropylamin als ein amphoteres grenzflächenaktives Agens;
b. ein anionisches grenzflächenaktives Agens;
c. wahlweise ein nicht-ionisches grenzflächenaktives Agens; und
d. mindestens zwei Konditionierer, ausgewählt aus:
1. einem kationischen Zellulose-Derivat;
2. einem kationischen Guar-Derivat; und
3. einem Homopolymer oder Co-Polymer eines kationischen Monomers, ausgewählt aus:
a. einem Monomer mit der Formel: 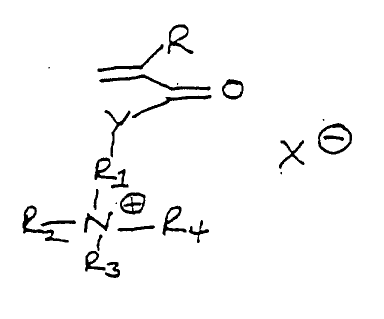 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder ein Alkyl, modifiziert durch eine Hydroxy-Gruppe mit 1 bis 22 Kohlenstoffatomen, sind; und X ein monovalentes Anion, ausgewählt aus Halogenid und Alkylsulfat mit 1 bis 4 Kohlenstoffatomen, ist; oder
b. Diallyldimethylammoniumchlorid.

17. Detergens-Zusammensetzung, die folgendes umfaßt:
a. ein amphoteres grenzflächenaktives Amidoalkylsultain-Agens der Formel: 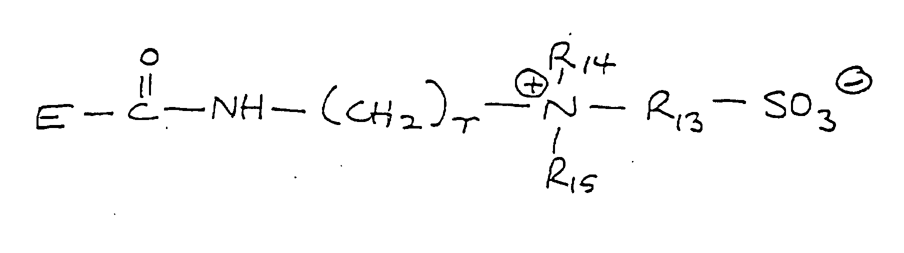 wobei E eine Alkyl-Gruppe oder Alkylen-Gruppe mit 7 bis 21 Kohlenstoffatomen ist; R₁₄ und R₁₅ jeweils unabhängig eine Alkyl-Gruppe oder eine Hydroxyalkyl-Gruppe mit 1 bis 4 Kohlenstoffatomen sind; r eine ganze Zahl von 2 bis 6 ist; und R₁₃ eine Alkylen- oder Hydroxyalkylen-Gruppe mit 2 bis 3 Atomen ist;
b. ein ionisches grenzflächenaktives Agens;
c. wahlweise ein nicht-ionisches grenzflächenaktives Agens;
d. mindestens ein Konditionierer-Paar, ausgewählt aus:
1. einem kationischen Zellulose-Derivat und einem kationischen Guar-Derivat;
2. einem kationischen Guar-Derivat und einem Homopolymer oder Co-Polymer von Diallyldimethylammoniumchlorid;
3. einem Homopolymer oder Co-Polymer eines Monomers mit der Formel: 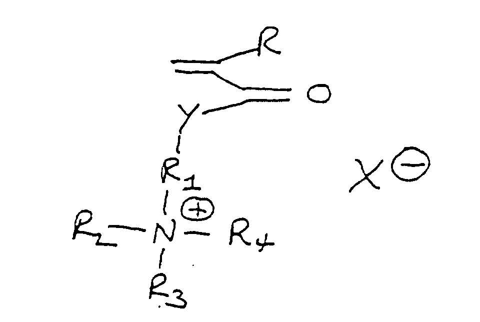 wobei R H oder CH₃ ist; Y O oder NH ist; R₁ eine Alkylen-Gruppe mit 2 bis 6 Kohlenstoffatomen ist; R₂, R₃ und R₄ jeweils unabhängig eine Alkyl-Gruppe oder Hydroxyalkyl-Gruppe mit 1 bis 22 Kohlenstoffatomen sind; und X ein monovalentes Anion, ausgewählt aus Halogenid und Alkylsulfat mit 1 bis 4 Kohlenstoffatomen, ist; und einem kationischen Guar-Derivat.

## Revendications

1. Composition détergente comprenant :
a) une partie de tensioactif comprenant :
1. un tensioactif non ionique ;
2. un tensioactif amphotère ; et
3. un tensioactif anionique et
b) une partie de conditionneur comprenant au moins une paire de polymères cationiques de conditionnement sélectionnés à partir de :
1. un dérivé de cellulose cationique et un dérivé de guar cationique;
2, un dérivé de guar cationique et un homopolymère ou un copolymère d'un monomère ayant la formule : 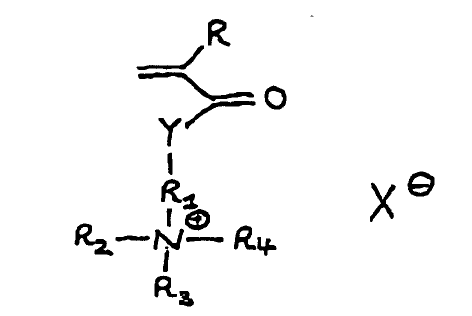 dans laquelle R est H ou CH₃, Y est O ou NH, R₁ est un groupe alkylène ayant de 2 à 6 atomes de carbone, R₂, R₃ et R₄ sont chacun indépendamment un groupe alkyle ou hydroxyalkyle ayant de 1 à 22 atomes de carbone, et X est un anion monovalent sélectionné à partir des halogénures et du sulfate d'alkyle ayant de 1 à 4 atomes de carbone ; ou
3. un homopolymère ou un copolymère d'un monomère cationique de chlorure de diallyldiméthylammonium et un dérivé cationique de guar.

2. Composition détergente et de conditionnement comprenant, sur la base du poids total de la composition détergente :
a) de 1 à 10 % de tensioactifs non ioniques comprenant :
1. un dérivé polyoxyéthylène d'un ester de polyol a) dérivé d'un acide gras contenant de 8 à 22 atomes de carbone et un polyol sélectionné à partir du sorbitol, du sorbitane, du glucose, du 1-méthyl glucoside, du polyglucose ayant en moyenne de 1 à 3 résidus de glucose, de la glycérine, du pentahérythritol et des mélanges de ceux-ci, b) contenant en moyenne de 10 à 120 unités d'oxyéthylène, et c) ayant en moyenne de 1 à 3 résidus d'acide gras par molécule de dérivé polyoxyéthylène d'ester de polyol,
2. un alkyl glucoside ayant un groupe alkyle contenant de 6 à 22 atomes de carbone et ayant de 1 à 6 résidus de glucose par molécule d'alkyl glucoside, ou
3. des mélanges de ceux-ci, et
b) de 0,5 à 10 % d'un ou de plusieurs tensioactifs amphotères amphocarboxylates de formule A - CONH (CH₂)ₓ N⁺ R₅R₆R₇, dans laquelle : A est un groupe alkyle ou alkényle ayant de 7 à 21 atomes de carbone ; x est un nombre entier allant de 2 à 6 ; R₅ est un groupe hydrogène ou carboxyalkyle contenant de 2 à 3 atomes de carbone ; R₆ est un groupe hydroxyalkyle contenant de 2 à 3 atomes de carbone de formule R₈ - O - (CH₂)ₙ CO₂⁻, dans laquelle R₈ est un groupe alkylène ayant de 2 à 3 atomes de carbone et n est 1 ou 2 ; et R₇ est un groupe carboxyalkylc contenant de 2 à 3 atomes de carbone,
c) de 0,5 à 10 % d'un ou de plusieurs tcnsioactifs amphotères de bétaïne sélectionnés parmi :
1. une alkyl bétaïne de formule B - N⁺ R₉R₁₀ (CH₂)ₚ CO₂⁻, dans laquelle : B est un groupe alkyle ou alkényle ayant de 8 à 22 atomes de carbone ; R₉ et R₁₀ sont chacun indépendamment un groupe alkyle ou hydroxyalkyle ayant de 1 à 4 atomes de carbone ; et p est 1 ou 2 ; ou
2. une amidoalkyl bétaïne de formule : D - CO - NH (CH₂)_{q} - N⁺ R₁₁R₁₂ (CH₂)ₘ CO₂⁻, dans laquelle D est un groupe alkyle ou alkényle ayant de 7 à 21 atomes de carbone ; R₁₁ et R₁₂ sont chacun indépendamment un groupe alkyle ou un groupe hydroxyalkyle ayant de 1 à 4 atomes de carbone ; q est un nombre entier de 2 à 6 ; et m est 1 ou 2 ;
d) de 1 à 10 % d'un ou de plusieurs tensioactifs anioniques d'alkyl éther sulfate de formule R' (OCH₂CH₂)ᵥ OSO₃X', dans laquelle R¹ est un groupe alkyle ou alkényle ayant de 7 à 22 atomes de carbone, X' est un ion métal alcalin, un ion de métal alcalinoterreux, un ion ammonium, un ion ammonium substitué avec de 1 à 3 substituants, chacun de ces substituants pouvant être identiques ou différents, et est un groupe alkyle ayant de 1 à 4 atomes de carbone, et v est un nombre entier de 1 à 6 ;
e) de 0,01 à 0,5 % de Polyquaternium - 10 ; et
f) de 0,01 à 0,5 % d'hydroxypropyltrimonium chlorure de guar ;
dans lequel les tensioactifs des points a) à d) sont présents dans une quantité allant de 5 % à 20 % sur la base du poids total de la composition.

3. Composition détergente selon la revendication 2, comprenant en outre de 0,25 % à 2,5 %, sur la base du poids total de la composition détergente, d'un agent de perlescence ou opacifiant comprenant un diester de : a) un acide gras ayant de 16 à 22 atomes de carbone ; et b) de l'éthylène glycol bu du propylène glycol.

4. Composition détergente selon la revendication 3, dans laquelle l'agent perlescent ou l'agent opacifiant est ajouté à la composition détergente sous la forme d'une dispersion aqueuse pré-formée stabilisée.

5. Composition détergente selon l'une quelconque des revendications 2 à 4, comprenant de 0,5 à 5 % d'un humectant comprenant la glycérine, sur la base du poids total de la composition.

6. Composition détergente selon l'une quelconque des revendications 2 à 5, comprenant en outre un épaississant comprenant un diester de : a) du polyéthylène glycol de formule : HO - (CH₂CH₂O)_{z} - H dans laquelle z est un nombre entier allant de 3 à 200 ; et b) est un acide gras contenant de 16 à 22 atomes de carbone, dans laquelle l'épaississant est présent dans une quantité suffisante pour fournir une composition détergente ayant une viscosité de Brookfield comprise entre 500 centipoises et 10.000 centipoises.

7. Composition détergente selon l'une quelconque des revendications 2 à 6, comprenant en outre, sur la base du poids total de la composition détergente, de 0,25 à 2,5 % d'un conditionneur à base de silicone composé de polydiméthylsiloxane, de polydiméthylcyclosiloxane, d'hexaméthyldisiloxane ou d'un mélange de celles-ci.

8. Composition détergente sclon la revendication 7, dans laquelle le conditionneur à base de siloxane comprend un composé silicone volatile ayant un point d'ébullition à pression atmosphérique qui est inférieur à environ 220°C.

9. Composition détergente selon l'une quelconque des revendications 2 à 8, comprenant en outre des additifs composés des colorants, des parfums, des conservateurs, des agents d'ajustement du pH et de mélanges de ceux-ci.

10. Composition détergente selon l'une quelconque des revendications 2 à 9, dans laquelle la composition détergente à un pH compris entre 5 et 7,5.

11. Composition détergente conditionnante comprenant, sur la base du poids total de la composition détergente :
A. de 5 à 20 % d'une partie de tensioactifs comprenant, sur la base du poids total de la composition :
1. de 2,0 à 8 % d'un tensioactif non ionique comprenant un dérivé polyoxyéthylène d'ester de polyol, un alkyl glucoside dans lequel le groupe alkyle contient de 8 à 14 atomes de carbone, ou un mélange de ceux-ci ;
2. de 1 à 6 % d'un tensioactif amphotère d'amidoalkyl sultaine ; et
3. de 1 à 6 % d'un tensioactif anionique d'alkyl éther sulfate ; et
B. de 0,01 à 1,0 % d'une partie conditionneur comprenant au moins une paire de conditionneurs polymères cationiques sélectionnés à partir de :
1. un dérivé de cellulose cationique et un dérivé de guar cationique;
2. un dérivé de guar cationique et un homopolymère ou un copolymère d'un monomère de formule 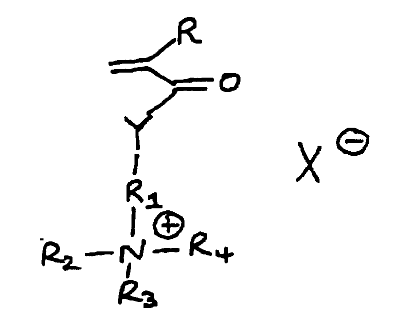 dans laquelle R est H ou CH₃, Y est O ou NH, R₁ est un groupe alkylène ayant de 2 à 6 atomes de carbone, R₂, R₃ et R₄ sont chacun indépendamment un groupe alkyle ou hydrcxyakyle ayant de 1 à 22 atomes de carbone et X est un anion monovalent sélectionné parmi l'halogénure et l'alkyle sulfate ayant de 1 à 4 atomes de carbone ; ou
3. un homopolymère ou un copolymère d'un monomère cationique de diallyldiméthylammonium chlorure et un dérivé de guar cationique.

12. Composition détergente conditionnante comprenant, sur la base du poids total de la composition détergente :
A. de 5 % à 20 % d'une partie de tensioactif comprenant, sur la base du poids total de la composition détergente:
1. de 0,1 à 8 % d'un tensioactif non ionique composé de :
a) un dérivé polyoxyéthylène d'ester de polyol ;
b) un alkyl glycoside, le groupe alkyle ayant de 8 à 14 atomes de carbone ; ou
c) des mélanges de ceux-ci ;
2. de 2 à 6 % d'un tensioactif amphotère carboxyalkyl alkyl polyamine de formule ; 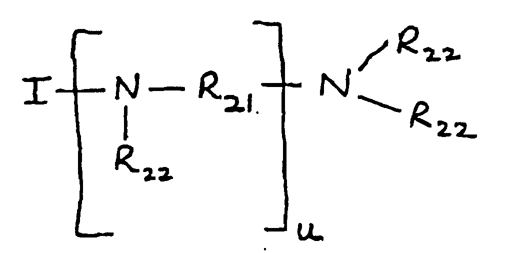 dans laquelle I est un groupe alkyle ou alkényle contenant de 8 à 22 atomes de carbone ; R₂₂ est un groupe carboxyalkyle ayant de 2 à 3 atomes de carbone ; R₂₁ est un groupe alkylène ayant de 2 à 3 atomes de carbone ; et u est un nombre entier allant de 2 à 4 ;
3. de 2 à 8 % d'un tensioactif anionique qui est un alkyl éther sulfosuccinate, un alkyl éther sulfate ou un mélange de ceux-ci, le groupe alkyle ayant de 8 à 14 atomes de carbone ; et
B. de 0,01 à 1,0 % d'une partie de conditionneur comprenant au moins deux polymère cationiques de conditionnement sélectionnés parmi :
1. un dérivé de cellulose cationique ;
2. un dérivé de guar cationique ;
3. un homopolymère ou un copolymère de monomère cationique sélectionné à partir de :
a. un monomère ayant la formule : 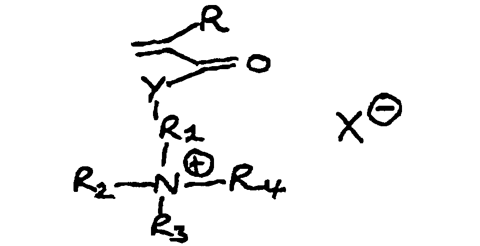 dans laquelle R est H CH₃, Y est O ou NH, R₁ est un groupe alkylène ayant de 2 à 6 atomes de carbone, R₂, R₃ et R₄ sont chacun indépendamment un groupe alkyle ou un groupe hydroxyalkyle ayant de 1 à 22 atomes de carbone, et X est un anion monovalent sélectionné à partir de l'halogénure et de l'alkyl sulfate ayant de 1 à 4 atomes de carbone, ou
b. du diallyldiméthylammonium chlorure

13. Composition détergente comprenant:
a. une carboxyméthyl cocopolypropylamine de sodium en tant que tensioactif amphotère ;
b. un tensioactif anionique, mis à part les tensioactifs anioniques du groupe composé de :
(i) un alkyl sulfate de formule R' - CH₂ OSO₃X' et
(ii) un alkylaryl sulfonate de folmule: 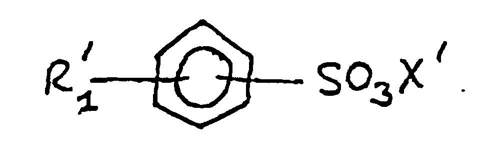
dans laquelle R' est un groupe alkyle ayant de 7 à 14 atomes de carbone, R'₁ est un groupe alkyle ayant de 1 à 12 atomes de carbone et X' est un ion de métal alcalin, un ion de métal alcalinoterreux, un ion ammonium ou un ion ammonium substitué avec de 1 à 3 substituants ; chacun de ces substituants pouvant être identiques ou différents et est un groupe alkyle ayant de 1 à 4 atomes de carbone ou un groupe hydroxyalkyle ayant de 2 à 4 atomes de carbone ; et
c. optionnellement un tensioactif non ionique,
à condition que si le tensioactif non ionique est omis et que si le tensioactif anionique est un alkyl sulfate éther de formule R' (OCH₂CH₂)ᵥ OSO₃X', alors v est supérieur ou égal à 3.

14. Composition détergente selon la revendication 13, dans laquelle le tensioactif anionique est un alkyl éther sulfosuccinate de formule : 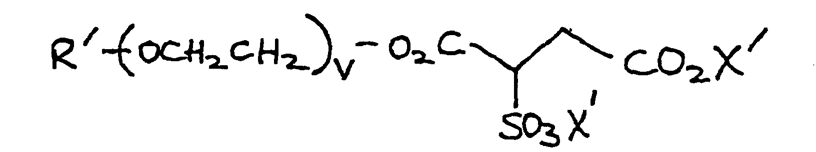 dans laquelle R' est un groupe alkyle ayant de 7 à 22 atomes de carbone, X' est un ion de métal alcalin, un ion de métal alcalinoterreux, un ion ammonium, un ion ammonium substitué avec de 1 à 3 substituants, chacun desdits substituants pouvant être identiques ou différents et est un groupe alkyle ayant de 1 à 4 atomes de carbone ou un groupe hydroxyalkyle ayant de 2 à 4 atomes de carbone, et v est un nombre entier de 1 à 6 ;
ou des mélanges de ceux-ci.

15. Composition détergente selon la revendication 13 ou 14, comprenant en outre au moins un conditionneur sélectionné à partir de :
1. un dérivé de cellulose cationique
2. un dérivé de guar cationique ; et
3. un homopolymère ou un copolymère d'un monomère cationique sélectionné à partir de :
a. un monomère avant la formule : 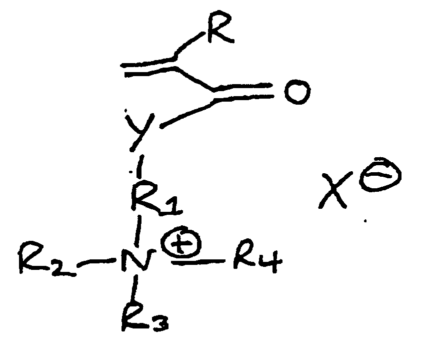 dans laquelle R est H ou CH₃, Y est O ou NH, R₁ est un groupe alkylène ayant de 2 à 6 atomes de carbone, R₂, R₃ et R₄ sont chacun indépendamment un groupe alkyle ou hydroxyalkyle ayant de 1 à 22 atomes de carbone, et X est un anion monovalent sélectionné à partir de l'halogénure et de l'alkyle sulfate ayant de 1 à 4 atomes de carbone, ou
b. du diallyldiméthylammonium chlorure.

16. Composition détergente comprenant :
a. une carboxyméthyl cocopolypropylamine de sodium en tant qu'un tcnsioactif amphotère ;
b. un tensioactif anionique ;
c. optionnellement un tensioactif non ionique ; et
d. au moins deux conditionneurs sélectionnés à partir de :
1. un dérivé de cellulose cationique ;
2. un dérivé de guar cationique ; et
3. un homopolymère ou un copolymère d'un monomère cationique sélectionné à partir de :
a. un monomère ayant la formule suivante : 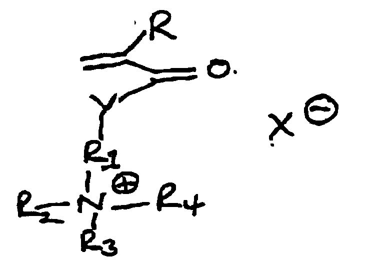 dans laquelle R est H ou CH₃, Y est O ou NH, R₁ est un groupe alkylène ayant de 2 à 6 atomes de carbone, R₂, R₃ et R₄ sont chacun indépendamment un groupe alkyle ou un groupe alkyle modifié avec un groupe hydroxy ayant de 1 à 22 atomes de carbone, et X est un anion monovalent sélectionné à partir de l'halogénure et de l'alkyl sulfate ayant de 1 à 4 atomes de carbone, ou
b. du diallyldiméthylammonium chlorure.

17. Composition détergente comprenant:
a. un tensioactif amphotère amidoalkyl sultaine de formule : 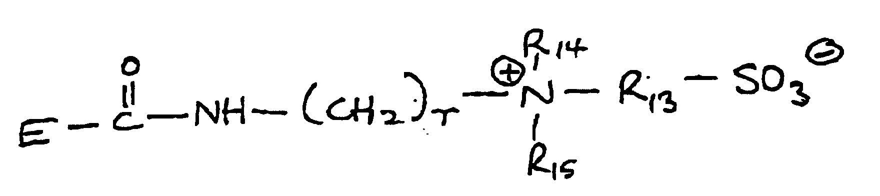 dans laquelle E est un groupe alkyle ou alkényle ayant de 7 à 21 atomes de carbone ; R₁₄ et R₁₅ sont chacun indépendamment un groupe alkyle ou un groupe hydroxyalkyle ayant de 1 à 4 atomes de carbone ; r est un nombre entier de 2 à 6 ; et R₁₃ est un groupe alkylène ou hydroxyalkylène ayant 2 ou 3 atomes de carbone ;
b. un tensioactif ionique ;
c. optionnellement un tensioactif non ionique ; et
d. au moins une paire de conditionneurs sélectionnés à partir de :
1. un dérivé de cellulose cationique et un dérivé de guar cationique ;
2. un dérivé de guar cationique et un homopolymère ou un copolymère de diallyldiméthylammonium chlorure ;
3. un homopolymère ou un copolymère d'un monomère ayant la formule : 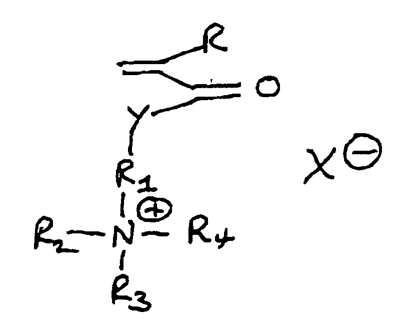 dans laquelle R est H ou CH₃, Y est O ou NH, R₁ est un groupe alkylène ayant de 2 à 26 atomes de carbone, R₂ R₃ et R₄ sont chacun indépendamment un groupe alkyle ou un groupe hydroxyalkyle ayant de 1 à 22 atomes de carbone et X est un anion monovalent sélectionné à partir de l'halogénure et de l'alkylsulfate ayant de 1 à 4 atomes de carbone ;
et un dérivé de guar cationique.
